# EUROPÄISCHE PATENTSCHRIFT

(11) **EP 3 356 324 B1**
(45) Veröffentlichungstag und Bekanntmachung des Hinweises auf die Patenterteilung: **05.10.2022**
(21) Anmeldenummer: 16770943.5
(22) Anmeldetag: 28.09.2016
(51) Int. Cl.: C07C 263/10

(54) **VERFAHREN ZUR HERSTELLUNG VON ISOCYANATEN**
METHOD FOR MAKING ISOCYANATES
PROCÉDÉ DE FABRICATION D'ISOCYANATES

(30) Priorität: 30.09.2015 EP 15187592
(43) Veröffentlichungstag der Anmeldung: 08.08.2018
(73) Patentinhaber: Covestro Intellectual Property GmbH & Co. KG, 51373 Leverkusen (DE)
(72) Erfinder: KNAUF, Thomas, 41542 Dormagen (DE); MANZEL, Dirk, 47447 Moers (DE); PLATHEN, Peter, 47829 Krefeld (DE); WERSHOFEN, Stefan, 41065 Mönchengladbach (DE)
(74) Vertreter: Levpat
(86) Internationale Anmeldenummer: PCT/EP2016/073036
(87) Internationale Veröffentlichungsnummer: WO 2017/055311

(56) Entgegenhaltungen:
- WO-A1-03/045900
- WO-A1-2013/029918
- DE-A1- 3 212 510
- DE-A1-102009 032 413

## Beschreibung

Die Erfindung betrifft ein kontinuierlich betriebenes Verfahren zur Herstellung von Isocyanaten durch Umsetzung der korrespondierenden Amine mit Phosgen, bei dem bei einer Änderung der angestrebten Produktionskapazität ausgehend von einem Anfangszustand mit einer bestimmten Produktionskapazität und endend in einem Endzustand mit einer anderen Produktionskapazität in der Übergangsperiode zwischen Anfangs- und Endzustand, die mindestens 0,50 Minuten und maximal 3 Stunden beträgt,
- der momentane Phosgenüberschuss zu dem Zeitpunkt der Übergangsperiode, bei dem mit der Änderung des Amin-Massenstroms begonnen wird, mindestens genau so groß ist wie und bevorzugt größer ist als der Phosgenüberschuss während des Zeitraums der Produktion mit der Produktionskapazität im Anfangszustand vor Beginn der Übergangsperiode, und
- der durchschnittliche Phosgenüberschuss während der Übergangsperiode größer ist als der Phosgenüberschuss während des Zeitraums der Produktion mit der Produktionskapazität im Anfangszustand vor Beginn der Übergangsperiode
wobei der Phosgenüberschuss während der Produktion mit der Produktionskapazität m_{1,Soll}(A) vor Beginn der Übergangsperiode gleich dem Phosgenüberschuss während der Produktion mit der Produktionskapazität m_{1, Soll} (E) nach dem Ende der Übergangsperiode ist.

Isocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen, wobei Phosgen im stöchiometrischen Überschuss eingesetzt wird. Die Umsetzung der Amine mit dem Phosgen kann sowohl in der Gasphase als auch in der Flüssigphase erfolgen, wobei die Reaktion diskontinuierlich oder kontinuierlich durchgeführt werden kann (W. Siefken, Liebigs Ann. 562, 75 - 106 (1949)). Verfahren zur Herstellung von organischen Isocyanaten aus primären Aminen und Phosgen sind bereits mehrfach beschrieben worden, siehe beispielsweise Ullmanns Encyklopädie der technischen Chemie, 4. Aufl. (1977), Band 13, S. 351 bis 353 sowie G. Wegener et. al. Applied Catalysis A: General 221 (2001), p. 303 - 335, Elsevier Science B.V. Es kommen dabei sowohl aromatische Isocyanate wie beispielsweise Methylendiphenyldiisocyanat (MMDI - "monomeres MDI"), Polymethylen-Polyphenylen-Polyisocyanat (ein Gemisch aus MMDI und höheren Homologen, PMDI, "polymeres MDI") oder Toluylendiisocyanat (TDI) als auch aliphatische Isocyanate wie z. B. Hexamethylendiisocyanat (HDI) oder Isophorondiisocyanat (IPDI) weltweit zum Einsatz.

Man unterscheidet generell zwischen zwei Arten der Verfahrensführung, nämlich der Reaktion in der Gasphase und der Reaktion in der Flüssigphase.

Die **Verfahrensführung in der Gasphase,** üblicherweise als Gasphasenphosgenierung bezeichnet, zeichnet sich dadurch aus, dass die Reaktionsbedingungen so gewählt werden, dass zumindest die Reaktionskomponenten Amin, Isocyanat und Phosgen, bevorzugt jedoch sämtliche Edukte, Produkte und Reaktionszwischenprodukte, bei den gewählten Bedingungen gasförmig sind.

Die **Verfahrensführung in der Flüssigphase,** üblicherweise als Flüssigphasenphosgenierung bezeichnet, zeichnet sich dadurch aus, dass die Reaktionsbedingungen so gewählt werden, dass zumindest die Reaktionskomponenten Amin, Roh-Isocyanat und Phosgen, bevorzugt jedoch sämtliche Edukte, Produkte und Reaktionszwischenprodukte, bei den gewählten Bedingungen flüssig (was in diesem Zusammenhang auch den Zustand eines in der Flüssigphase physikalisch gelösten Gases umfasst) in einem geeigneten Lösungsmittel vorliegen und das Lösungsmittel vom Roh-Isocyanat abgetrennt wird und gereinigt oder auch ungereinigt in den Reaktionskreislauf zurückgeführt wird.

Für die Vermischung des Amins mit dem Phosgen in der Flüssigphasenphosgenierung kann ein dynamischer oder statischer Mischer benutzt werden, in der Gasphasenphosgenierung empfiehlt sich der Einsatz einer Mischdüse. Wichtig ist eine schnelle und gute Vermischung der beiden Einsatzstoffe, da das entstehende Isocyanat bei einer schlechten Durchmischung der Einsatzstoffe mit dann lokal überschüssig auftretendem Amin zu Harnstoff bzw. anderen störenden, hochviskosen und festen Nebenprodukten reagiert. Dadurch entstehen Anbackungen am Equipment und Verstopfungen (z. B. in den Rohrleitungen), die zu unerwünschten Reinigungsstillständen und damit zu schlechteren Laufzeiten der Anlage, sprich zu einer suboptimalen Wirtschaftlichkeit, führen. Deswegen lag der Fokus in der Isocyanat herstellenden Industrie in der jüngeren Vergangenheit auf der **Optimierung der Vermischung der Einsatzstoffe,** wie man auch an der Vielzahl an Patentveröffentlichungen ablesen kann. Beispielhaft seien hier die folgenden Patentanmeldungen aufgeführt: CN 102527312 A, WO 2013/048873 A1, WO 2013/060836 A1, EP 2 077 150 A1, EP-A-1 555 258, WO 2006/108740 A1, WO 2010/015667 A1, oder EP-A-1 526 129.

Die moderne großtechnische Herstellung von Polyisocyanaten erfolgt kontinuierlich, und die Reaktionsführung erfolgt bevorzugt als adiabate Phosgenierung, wie z. B. in EP 1 616 857 A1 beschrieben. Unerwünschte Ablagerungen und Nebenprodukte im Reaktor werden *durch richtige Wahl von Reaktionstemperatur und Druck* vermieden. Im Mischraum ist ein molarer Überschuss von Phosgen zu den primären Aminogruppen zu gewährleisten. Eine dreistufige Phosgenierstraße ist in EP 1 873 142 A1 beschrieben, bei der der Druck zwischen der ersten Stufe eines Mischers und der zweiten Stufe eines ersten Phosgenierreaktors gleich bleibt oder ansteigt und in der dritten Stufe, einem Apparat zur Phosgenabtrennung, der Druck niedriger ist als in der zweiten Stufe.

WO 2013/029918 A1 (fortan WO '918) beschreibt ein Verfahren zur Herstellung von Isocyanaten durch Umsetzung der korrespondierenden Amine mit Phosgen, das bei unterschiedlichen Auslastungen der Anlage ohne Probleme durchgeführt werden kann. Insbesondere sollen auch beim Betreiben der Anlage im Teillastbereich das Mischen und/oder die Reaktion in dem jeweils optimierten Verweilzeitfenster erfolgen, indem das Verhältnis von Phosgen zu Amin erhöht wird oder ein oder mehrere inerte Stoffe dem Phosgen- und/oder Aminstrom zugesetzt werden. Das erfindungsgemäße Verfahren soll es ermöglichen, eine bestehende Anlage bei unterschiedlichen Auslastungen mit gleichbleibender Produkt- und Verfahrensqualität zu betreiben. Dies soll die Anschaffung mehrerer Anlagen mit unterschiedlichen Nennkapazitäten einsparen. Es werden folgende Feststellungen getroffen: Sowohl bei der GasphasenPhosgenierung als auch bei der Flüssigphasen-Phosgenierung sind das Mischen der Edukte sowie die Verweilzeit des Reaktionsgemischs in den entsprechenden Reaktionsräumen kritische Parameter. Die Anlagen zur Herstellung von Isocyanaten durch Phosgenierung von Aminen müssen daher an die speziellen Anforderungen hinsichtlich schneller Durchmischung der Eduktströme und enge Verweilzeitfenster angepasst sein. Anlagen zur Phosgenierung von Aminen werden dabei im Wesentlichen an die maximalen Mengenströme bzw. an die jeweilige Nennkapazität ausgelegt. Dies bedeutet, sowohl Mischorgane, wie Düsen, als auch die Reaktionsräume, beispielsweise Verweilzeitreaktoren, arbeiten bei der Nennkapazität im optimalen Bereich mit optimierter Ausbeute, Reinheit der Produkte etc. Ist die Anlage jedoch nicht voll ausgelastet, d. h., dass sie nur bei einem Teil der Nennkapazität betrieben wird, ändern sich beispielsweise die Verweilzeiten, *und die Anlage arbeitet infolgedessen nicht mehr im optimalen Bereich.* Dies ist beispielsweise bei An- und Abfahrvorgängen, Teilauslastung der Anlage oder Störungen in der Anlage der Fall. In diesen Fällen verminderter Produktionskapazität arbeiten sowohl die Mischorgane als auch die Verweilzeitreaktoren nicht im optimalen Bereich. Die Folge sind Ausbeuteverluste, Probleme mit Anbackungen im Equipment und/oder Qualitätseinbußen. Um die vorgenannten Probleme zu vermeiden, schlägt die WO '918 vor, dass auf eine ausreichend schnelle Vermischung der Reaktanden geachtet werden muss. Methoden zur Durchführung kurzer Mischzeiten sind im Prinzip bekannt. In den Mischeinheiten können Mischaggregate mit dynamischen oder statischen Mischern eingesetzt werden. Bevorzugt werden in den Mischeinheiten ein oder mehrere statische Mischorgane eingesetzt. Als statische Mischorgane geeignet sind beispielsweise aus der Verbrennungstechnik bekannte Düsen, Glattstrahldüsen oder Venturidüsen, sowie Lavaldüsen. Die WO '918 schlägt als eine besonders vorteilhafte Ausführung eines statischen Mischorgans ein solches Mischorgan vor, wie es in WO 2010/015667 A1 beschrieben ist. Als dynamische Mischer können beispielsweise in den Mischeinheiten angeordnete Rotor/Statorsysteme eingesetzt werden. Bevorzugt werden gemäß WO '918 statische Mischorgane, insbesondere Düsen eingesetzt. Das Verfahren gemäß WO '918 löst diese Probleme (Ausbeuteverluste, Foulingprobleme und/oder Qualitätseinbußen) allerdings auf Kosten des Einsatzes von erhöhten Mengen an Lösungsmittel und/oder an erhöhten Mengen an Phosgen und nicht durch die Verwendung geeigneter Düsen. Dies bedeutet, dass bei Teilauslastung der Anlage die Verweilzeit in den Reaktoren und Apparaten der Anlage im Vergleich zum bestimmungsgemäßen Betrieb der Anlage bei Nennkapazität gleichgehalten oder annähernd gleichgehalten wird. Daraus ergeben sich gravierende Nachteile wie z. B. ein höherer spezifischer und gegebenenfalls absoluter Phosgen-Hold-Up bzw. eine Verwendung einer erhöhten Menge an Lösungsmittel bei einer Produktionsteilauslastung. Damit einher geht natürlich auch die höhere energetische Belastung der Aufarbeitung des Roh-Isocyanates, wobei mehr als nötig an Lösungsmittel destilliert und mehr als nötig an Phosgen zurückgewonnen, sprich kondensiert werden muss. Dadurch leidet die Wirtschaftlichkeit des Verfahrens zusätzlich zu der bereits geringeren Auslastung.

Die in der WO'918 diskutierte Problematik der optimalen Fahrweise bei unterschiedlichen Auslastungszuständen gibt Anlass zu einigen grundsätzlichen Überlegungen:
Sowohl das Betreiben einer Produktionsanlage im Bereich der Nennkapazität (auch als "Nennlast" bezeichnet) wie auch im Bereich verminderter Produktionskapazität (auch als "Teillastbereich" bezeichnet) ist jeweils als ein **stationärer Zustand** in dem Sinne zu betrachten, dass die Mengenströme an Reaktanden (Amin, Phosgen, ggf. Verdünnungsmittel), sobald einmal eingestellt, *konstant bleiben.* Um von einem stationären Zustand (beispielsweise Produktion bei Nennkapazität) in einen anderen stationären Zustand (bspw. Produktion bei 75 % der Nennkapazität) zu wechseln, wird jedoch zwangsläufig jedes Mal ein Übergangszustand durchlaufen, in welchem die Mengenströme an Reaktanden ständigen Veränderungen unterworfen sind, an deren Ende der neue stationäre Zustand steht. In solchen Übergangszuständen ändern sich daher die Mengenströme, entweder kontinuierlich oder in diskreten Intervallen. Auf die detaillierte Ausgestaltung solcher Übergangszustände geht die WO '918 nicht ein. Vielmehr ist die WO '918 nur mit dem optimalen Betreiben des Verfahrens in den stationären Zuständen vor und nach einem Übergangszustand befasst.

Die Offenlegungsschrift DE 10 2009 032413 A1 befasst sich mit einem Verfahren zur Herstellung von Isocyanaten in der Gasphase, bei dem durch eine besondere Verfahrensführung in der Aufarbeitung des in der Phosgenierung entstehenden Gasstroms enthaltend Chlorwasserstoff und Phosgen die Phosgenrückgewinnungsausbeute erhöht wird. Auch diese Schrift befasst sich nicht mit Übergangszuständen zwischen zwei Betriebszuständen mit unterschiedlicher Last.

Der Patentanmeldung WO 03/045900 A1 liegt die Zielsetzung zugrunde, ein Verfahren zur Herstellung von Isocyanaten durch Phosgenierung in der Gasphase bereitzustellen, durch das sowohl eine hohe Wärmeaustauschfläche als auch trotz nicht vollständig zu vermeidender Feststoffbildung eine möglichst lange Betriebszeit der Produktionsanlage, insbesondere einer großtechnischen Produktionsanlage, erreicht werden soll. Zu diesem Zweck wird vorgeschlagen, die Reaktion in einem nicht zylindrischen Reaktionskanal durchzuführen. Auch diese Schrift befasst sich nicht mit Übergangszuständen zwischen zwei Betriebszuständen mit unterschiedlicher Last.

Die Offenlegungsschrift DE 32 12 510 A1 beschreibt ein zweistufiges Flüssigphasenverfahren zur kontinuierlichen Herstellung eines organischen Isocyanats. Auch diese Schrift befasst sich nicht mit Übergangszuständen zwischen zwei Betriebszuständen mit unterschiedlicher Last.

Es bestand daher Bedarf an weiteren Verbesserungen in der Herstellung von Isocyanaten. Insbesondere bestand Bedarf an einem Verfahren zur Herstellung von Isocyanaten durch Umsetzung der korrespondierenden Amine mit Phosgen, das bei unterschiedlichen Auslastungen ohne die vorstehend beschriebenen Nachteile durchgeführt werden kann, und das bei einem tatsächlichen Lastwechsel in der nicht-stationären Phase so geführt werden kann, dass es nicht zu Anbackungen und Niederschlägen im Equipment kommt und die eingesetzte Produktions-Anlage auch nach mehreren Lastwechseln reibungslos und mit guter Produktqualität über einen langen Zeitraum betrieben werden kann. Beim Betreiben der Produktions-Anlage in den stationären Phasen (Nennkapazität- oder Teillastbereich) soll das Verhältnis von Phosgen zu Amin und auch die Konzentration der Einsatzstoffe Phosgen und Amin in Lösungsmittel bzw. Inertgasen gleich gehalten werden und das Mischen und/oder die Reaktion in dem daraus resultierenden Verweilzeitfenster erfolgen.

Diesem Bedarf Rechnung tragend ist ein Gegenstand der vorliegenden Erfindung ein **kontinuierlich betriebenes Verfahren zur Herstellung eines Isocyanats (1)** mit einer angestrebten Produktionskapazität an Isocyanat **(1),** ausgedrückt als Massenstrom, von m_{1,Soll}, wobei mindestens einmal ein Wechsel der angestrebten Produktionskapazität m_{1, Soll} (ein sog. "Lastwechsel") durchgeführt wird, umfassend die Umsetzung des zu dem Isocyanat **(1)** korrespondierenden Amins **(2)** mit Phosgen **(3)** in einem Reaktionsraum, bei dem
(i) das Amin **(2)** dem Reaktionsraum mit einem Massenstrom m₂ und Phosgen **(3)** dem Reaktionsraum mit einem Massenstrom m₃ zugeführt werden, wobei
(ii) m₂ und m₃ stets so gewählt werden, dass Phosgen **(3)** im Überschuss bezogen auf die primären Aminogruppen des Amins **(2)** vorliegt, wobei
(iii) bei dem mindestens einen Wechsel der angestrebten Produktionskapazität m_{1, Soll}, welcher von einem Ausgangszustand m_{1, Soll} (A) ≠ 0 mit einem korrespondierenden Amin-Massenstrom m₂, _{Soll} (A) und einem unter Beachtung von (ii) ausgewählten Phosgen-Massenstrom m₃, _{Soll} (A) auf einen Endzustand m_{1, Soll} (E) ≠ 0 mit einem korrespondierenden Amin-Massenstrom m₂, _{Soll} (E) und einem unter Beachtung von (ii) ausgewählten Phosgen-Massenstrom m₃, _{Soll} (E) durchgeführt wird, wobei der Wechsel der angestrebten Produktionskapazität m_{1, Soll} um einen Wert erfolgt, der im Bereich von 10% bis 90%, bezogen auf m_{1, Soll} (A), liegt, und wobei der Wechsel der angestrebten Produktionskapazität m_{1, Soll} von dem Ausgangszustand m_{1, Soll} (A) ≠ 0 auf den Endzustand m_{1, Soll} (E) ≠ 0 in einer Übergangsperiode t_{Ü} erfolgt, die mindestens 0,50 Minuten und maximal 3 Stunden beträgt,
   die Massenströme an Amin **(2)** m₂ und Phosgen **(3)** m₃ während der Übergangsperiode zwischen Anfangs- und Endzustand so geändert werden, dass
   (iii-1) der momentane Phosgenüberschuss zu dem Zeitpunkt der Übergangsperiode, bei dem mit der Änderung des Massenstroms m₂ von m₂, _{Soll} (A) auf m_{2, Soll} (E) hin begonnen wird, mindestens genau so groß ist wie und bevorzugt größer ist als der Phosgenüberschuss während des Zeitraums der Produktion mit der Produktionskapazität m_{1, Soll} (A) vor Beginn der Übergangsperiode, und dass
   (iii-2) der durchschnittliche Phosgenüberschuss während der Übergangsperiode größer ist als der Phosgenüberschuss während des Zeitraums der Produktion mit der Produktionskapazität m_{1, Soll} (A) vor Beginn der Übergangsperiode,
   wobei der Phosgenüberschuss während der Produktion mit der Produktionskapazität m_{1, Soll} (A) vor Beginn der Übergangsperiode gleich dem Phosgenüberschuss während der Produktion mit der Produktionskapazität m_{1, Soll} (E) nach dem Ende der Übergangsperiode ist.

Die vorliegende Erfindung befasst sich mit Änderungen der angestrebten Produktionskapazität (d. h. es gilt mi, _{Soll} (A) ≠ m_{1, Soll} (E)) *während des kontinuierlichen Betriebs,* d. h. ausgehend von einem bestimmten Zustand *der Produktion* des Isocyanats (**1**) (d. h. m_{1, Soll} (A) # **0)** und endend in einem anderen Zustand *der Produktion* des Isocyanats (**1**) (m_{1, Soll} (E) ≠ **0).** Mit anderen Worten: Das erfindungsgemäße Verfahren umfasst in allen Ausführungsformen mindestens einen Wechsel (eine Änderung) der angestrebten Produktionskapazität (d. h. es gilt m_{1, Soll} (A) ≠ m_{1, Soll} (E)), und zwar insbesondere einen Wechsel der angestrebten Produktionskapazität um einen Wert, der im Bereich von 10% bis 90%, besonders bevorzugt im Bereich von 20 % bis 80 %, ganz besonders bevorzugt im Bereich von 30 % bis 70 %, jeweils bezogen auf den Ausgangswert m₁, _{Soll} (A), liegt. Bei einem Ausgangswert von m₁, _{Soll} (A) von beispielsweise 150 kg/h entspricht demnach eine Änderung der angestrebten Produktionskapazität um 30 % bezogen auf m_{1, Soll} (A) entweder einer Senkung von m_{1, Soll} auf 105 kg/h oder einer Steigerung von m_{1, Soll} auf 195 kg/h. Solche Änderungen der Produktionskapazität werden auch als *Lastwechsel* bezeichnet. Lastwechsel in diesem Sinne sind zu unterscheiden von An- und Abfahrvorgängen, bei denen eine nicht in Betrieb befindliche Produktionsanlage (d.h. m_{1, Soll} (A) = **0)** in Betrieb genommen (d.h. m_{1, Soll} (E) ≠ 0) bzw. eine in Betrieb befindliche Produktionsanlage (d.h. m_{1, Soll} (A) ≠ 0) außer Betrieb genommen (d. h. m_{1, Soll} (E) = **0)** wird. Die vorliegende Erfindung befasst sich damit, wie **zwischen** den beiden stationären Zuständen vor- und nach dem Lastwechsel, also **in der Übergangsperiode** zwischen Anfangs- und Endzustand, in vorteilhafter Weise zu verfahren ist. Mit anderen Worten: Verfahren, bei denen ein Isocyanat **(1)** stets mit der - im Rahmen betriebsüblicher Schwankungen von ± 2,5 %, bevorzugt ± 1,0 %, des Soll-Werts - gleichen angestrebten Produktionskapazität m_{1, Soll} hergestellt wird und bei denen die *momentane* Produktionskapazität m₁ nur bei der Inbetriebnahme (Zeitraum zwischen m₁ = 0 und m₁ = m_{1, Soll}) und bei der Außerbetriebnahme (Zeitraum zwischen m₁ = m_{1, Soll} und m₁ = 0) von dieser angestrebten Produktionskapazität (zwangsläufig) abweicht, es also eine Übergangsperiode im zuvor beschriebenen Sinne gar nicht gibt, sind nicht Gegenstand der vorliegenden Erfindung.

Im *Reaktionsraum* findet die Phosgenierung des Amins **(2)** zum korrespondierenden Isocyanat (**1**) statt. Der Reaktionsraum beginnt dort, wo Amin (**2**) und Phosgen **(3)** erstmalig aufeinandertreffen. Dies kann in einer Mischeinrichtung sein, der eine Verweilzeiteinrichtung zur Umsatzvervollständigung nachgeschaltet ist. In diesem Fall bilden Mischeinrichtung und Verweilzeiteinrichtung zusammen den Reaktionsraum.

Das herzustellende Isocyanat (**1**) wird mit einer *angestrebten Produktionskapazität m_{1, Soll}* (angegeben als Massenstrom, also als "pro Zeiteinheit hergestellte Masse an Isocyanat", z. B. in *kg Isocyanat pro Stunde)* produziert. Die angestrebte Produktionskapazität ist innerhalb der durch die Randbedingungen (wie z. B. der Größe der vorhandenen Apparate) einer vorhandenen Produktionsanlage festgelegten technischen Grenzen variabel und wird abhängig von den Marktanforderungen eingestellt. Dieser angestrebten Produktionskapazität entspricht ein *Amin-Massenstrom m₂*, *_{Soll}* (ebenfalls angegeben als Massenstrom, z. B. in *kg Amin pro Stunde),* welcher unter der Annahme 100%iger Ausbeute aus der angestrebten Produktionskapazität berechnet wird. Der angestrebte Phosgen-Massenstrom *m₃, _{Soll}* (ebenfalls angegeben als Massenstrom, z. B. in *kg Phosgen pro Stunde)* wird stets so gewählt, dass Phosgen *im Überschuss bezogen auf die primären Aminogruppen des Amins (2)* vorliegt. Theoretisch reagiert ein Mol Phosgen mit einem Mol primärer Aminogruppen zu einem Mol Isocyanatgruppen. Beispielsweise werden demnach bei einem Diamin als zu phosgenierendem Amin **(2)** mehr als zwei Mole Phosgen pro Mol Diamin eingesetzt. Die gegenüber der theoretisch erforderlichen Menge an Phosgen zusätzlich eingesetzte Menge an Phosgen wird als *Phosgenüberschuss* bezeichnet. Die Massenströme m₂ und m₃ beziehen sich im Rahmen der vorliegenden Erfindung jeweils auf Amin **(2)** und Phosgen **(3)** *als solches.* Werden Amin **(2)** und Phosgen **(3)** mit einem Lösungsmittel oder inertem Gas zu einem Gemisch **(20)** enthaltend Amin **(2)** und einem Gemisch **(30)** enthaltend Phosgen **(3)** verdünnt (was bevorzugt ist), so ist für die Bestimmung des Phosgenüberschusses selbstverständlich der darin jeweils enthaltende Anteil an Amin **(2)** und Phosgen **(3)** maßgeblich.

Die Produktion des Isocyanats **(1)** bei einer bestimmten angestrebten Produktionskapazität (bspw. bei der Nennkapazität der Produktionsanlage) m_{1, Soll} (A) wird für einen Zeitraum *t_{A}* betrieben *(Ausgangszustand).* Während dieses Zeitraums *t_{A}* ist m₂ = m₂, _{Soll} (A). Der Phosgen-Massenstrom m₃ während dieses Zeitraums *t_{A}* wird auf einen bestimmten Wert m_{3, Soll} (A) eingestellt.

Nun soll die Produktionskapazität verändert werden (bspw. auf 75 % der Nennkapazität [entsprechend einer Änderung, bezogen auf m_{1, Soll} (A), um 25 %] als Folge gesunkener Nachfrage nach dem produzierten Isocyanat). Der neue angestrebte Zustand entspricht dem *Endzustand.* Der Zeitraum der Produktion bei diesem neuen Zustand wird in der folgenden Diskussion auch als *t_{E}* bezeichnet. (Falls zu einem späteren Zeitpunkt ein erneuter Lastwechsel geplant sein sollte, wäre dieser Zeitraum *t_{E}* "aus der Perspektive dieses neuen Lastwechsels" als *t_{A}* anzusehen. Für die Diskussion ist dies jedoch unerheblich; aus Gründen der Vereinfachung der Diskussion wird daher im Folgenden stets von einem Lastwechsel mit einem vorangehenden und einem nachfolgenden stationären Zustand während der Zeiträume *t_{A}* bzw. *t_{E}* ausgegangen.) Zwischen dem Ende des Anfangszustands (im gewählten Beispiel der Produktion bei Nennkapazität) und dem Erreichen des Endzustands (im gewählten Beispiel der Produktion bei 75 % Nennkapazität) liegt eine *Übergangsperiode t_{Ü}.* Diese Übergangsperiode *t_{Ü}* beginnt zu dem Zeitpunkt, zu dem der Massenstrom m₂ und/oder m₃ vom jeweils eingestellten Soll-Wert m_{2, Soll} (A) bzw. m_{3,Soll} (A) erstmals zum Zwecke einer Änderung der Produktionskapazität erhöht oder reduziert wird. Dieser Zeitpunkt wird im Folgenden mit *to* bezeichnet. Beginnt die Änderung der Massenströme m₂ und m₃ vom jeweils eingestellten Soll-Wert m_{2, Soll} (A) bzw. m_{3, Soll} (A) auf den jeweils angestrebten Endwert hin zu unterschiedlichen Zeitpunkten, was bevorzugt ist, so wird der Zeitpunkt des Beginns derjenigen Massenstrom-Änderung, die früher eingeleitet wird, mit *to* und der Zeitpunkt des Beginns derjenigen Massenstrom-Änderung, die später eingeleitet wird, mit *t₂* bezeichnet (vgl. auch die Abbildungen). Insofern Abweichungen des Phosgenmassenstroms m₃ von m_{3, Soll} (A) lediglich Schwankungen (seien sie beabsichtigt oder unbeabsichtigt) des Phosgenüberschusses während des Zeitraums *t_{A}* widerspiegeln, markieren solche Veränderungen noch nicht den Beginn der Übergangsperiode *t_{Ü}.* Die Übergangsperiode *t_{Ü}* endet zu dem Zeitpunkt, zu dem die Massenströme m₂ und m₃ ihren jeweils angestrebten Soll-Wert m_{2, Soll} (E) bzw. m_{3, Soll} (E) erreicht haben. Dieser Zeitpunkt wird im Folgenden mit *t₁* bezeichnet.

Erfindungswesentlich ist nun **zum einen,** dass *der momentane Phosgenüberschuss zu dem Zeitpunkt der Übergangsperiode, bei dem mit der Änderung des Massenstroms m₂ von m*_{*2,* Soll} *(A) auf m₂,* _{Soll} *(E) hin begonnen wird, mindestens genau so groß ist wie und bevorzugt größer ist als der Phosgenüberschuss während des Zeitraums der Produktion mit der Produktionskapazität m_{1,Soll}(A) vor Beginn der Übergangsperiode* **(Merkmal (iii-1)).** Der Phosgenüberschuss lässt sich bei einem gegebenen Amin einfach aus den bekannten Werten der beiden Massenströme m₂ und m₃ berechnen. Der *Phosgenüberschuss während des Zeitraums der Produktion mit der Produktionskapazität m_{1, Soll} (A)* ergibt sich somit in einfacher Weise aus den beiden Strömen m_{2,Soll}(A) und m_{3, Soll} (A). Sollte (was nicht bevorzugt ist) der momentane Phosgenmassenstrom m₃ während des Zeitraums *t_{A}* Schwankungen unterworfen sein und infolgedessen vom angestrebten Wert m_{3, Soll} (A) zeitweise signifikant abweichen, wird zum Zwecke der Einstellung des *momentanen Phosgenüberschusses zu dem Zeitpunkt der Übergangsperiode, bei dem mit der Änderung des Massenstroms m₂ von m*_{*2,* Soll} *(A) auf m*_{*2,* Soll} *(E) hin begonnen wird,* der unter Verwendung des *durchschnittlichen* Phosgenmassenstroms m₃ während des Zeitraums *t_{A}* ermittelte *Phosgenüberschuss während des Zeitraums der Produktion mit der Produktionskapazität m*_{*1,* Soll} *(A) vor Beginn der Übergangsperiode* zugrunde gelegt.

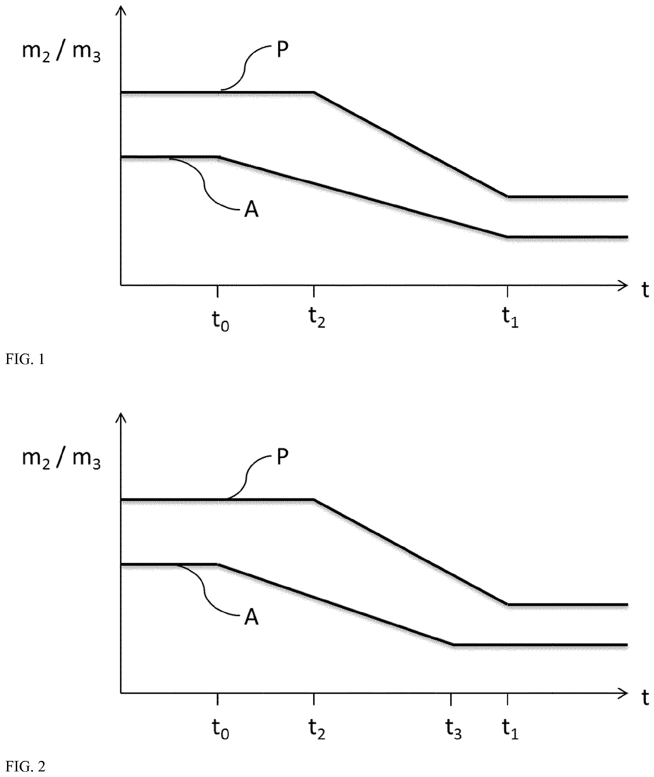

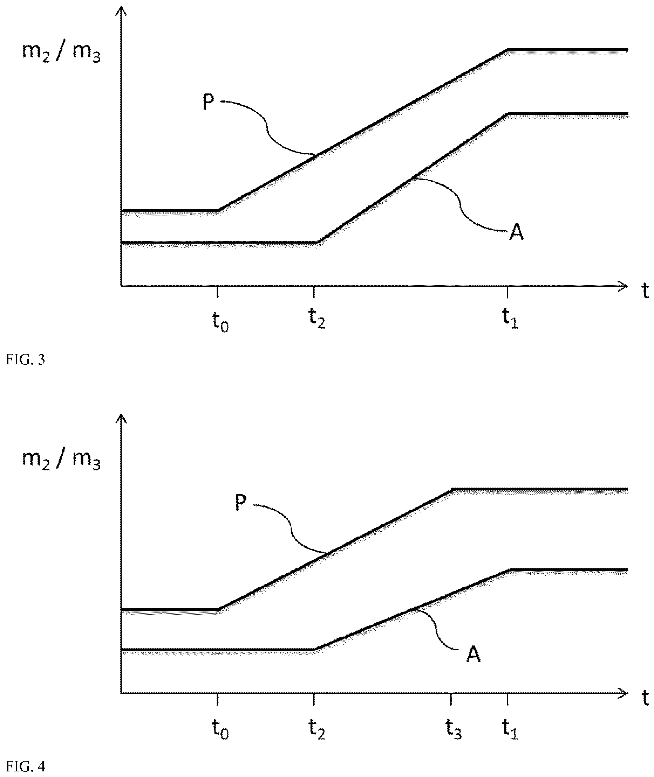

Erfindungswesentlich ist **zum anderen,** dass der *durchschnittliche Phosgenüberschuss während der Übergangsperiode größer ist als der Phosgenüberschuss während des Zeitraums der Produktion mit der Produktionskapazität m_{1,Soll}(A) vor Beginn der Übergangsperiode* **(Merkmal (iii-2)).** Während der Übergansperiode *t_{Ü}* ist der momentane Phosgenüberschuss veränderlich. Dies kann daran liegen, dass m₂ und m₃ zu unterschiedlichen Zeitpunkten erstmals zum Zwecke eines Lastwechsels erhöht oder erniedrigt werden (vgl. die Zeichnungen **FIG. 1** bis **FIG. 4****)** und / oder daran, dass die Funktion m₃(t) zwischen *to* und *t₁* insgesamt stärker abfällt als m₂(t), wie schematisch in **FIG.1** und **FIG. 2** dargestellt, und / oder daran, dass die Funktion m₃(t) zwischen *to* und *t₁* insgesamt steiler ansteigt als m₂(t), wie schematisch in **FIG. 3** und **FIG. 4** dargestellt. Wesentlich ist nun, dass der *durchschnittliche Phosgenüberschuss während der Übergangsperiode* (also im Zeitraum *t_{Ü}* beginnend bei *t₀* und endend bei *t₁*) größer ist als *der Phosgenüberschuss während des Zeitraums der Produktion mit der Produktionskapazität m*_{*1,* Soll} *(A) vor Beginn der Übergangsperiode.* In Bezug auf Letzteren gilt selbstverständlich das zuvor zu Merkmal (iii-1) Ausgeführte ebenso. Der Durchschnitt des Phosgenüberschusses während der Übergangsperiode kann in einfacher Weise rechnerisch ermittelt werden, z. B. aus den im Prozessleitsystem hinterlegten Massenströmen für Phosgen und Amin.

Nachfolgend werden besondere Ausführungsformen der Erfindung näher erläutert. Verschiedene Ausführungsformen sind dabei beliebig miteinander kombinierbar, solange sich für den Fachmann aus dem Zusammenhang nicht das Gegenteil ergibt.

Wie eingangs bereits erwähnt, befasst sich die vorliegende Erfindung mit Änderungen der angestrebten Produktionskapazität *während des kontinuierlichen Betriebs,* d. h. mit sog. *Lastwechseln.* Wie dem Fachmann bekannt ist, wird eine Produktionsanlage für die Herstellung von Isocyanaten **(1)** für eine bestimmte angestrebte Nennkapazität ausgelegt. Die *Nennkapazität* bedeutet dabei die Menge des pro Zeiteinheit produzierten Zielprodukts einer chemischen Anlage, für die die Anlage ausgelegt bzw. dimensioniert wurde. Diese Nennkapazität kann bei heutigen Anlagengrößen in der Größenordnung von mehreren hunderttausend Tonnen des angestrebten Isocyanats **(1)** pro Jahr liegen. Der Betrieb bei der Nennkapazität gibt daher auch die Mengenströme (Mengen pro Zeiteinheit) vor, die zur Erzielung der Nennkapazität einer Anlage eingesetzt werden. Bei der Herstellung von Isocyanaten **(1)** aus den entsprechenden Aminen **(2)** und Phosgen **(3)** bedeutet das, dass eine Produktionsanlage für die Produktion einer bestimmten Menge Isocyanat in einer bestimmten Zeitspanne ausgelegt ist (m_{1, Soll, Nenn}) und dafür dem Reaktionsraum bestimmte Massenströme Amin (m_{2, Soll, Nenn}) und Phosgen (m_{3,Soll,Nenn}) zugeführt werden müssen. Im Rahmen der Erfindung bezieht sich der Begriff "unterhalb der Nennkapazität" (oder auch "Teillast") auf das bzw. die eingesetzten Amine **(2);** d. h. "Betrieb unterhalb der Nennkapazität" bedeutet, dass der eingesetzte Mengenstrom Amin m₂ kleiner als der Mengenstrom Amin ist, der für den Betrieb bei Nennkapazität einer Produktionsanlage vorgesehen ist (m_{2, Soll, Nenn}).

Das erfindungsgemäße Verfahren eignet sich insbesondere für solche Lastwechsel, bei denen der Anfangs- oder Endzustand im Bereich von
- der Nennkapazität der Produktionsanlage bis zu 20 % der Nennkapazität der Produktionsanlage,
- bevorzugt von der Nennkapazität der Produktionsanlage bis zu 30 % der Nennkapazität der Produktionsanlage und
- besonders bevorzugt von der Nennkapazität der Produktionsanlage bis zu 40 % der Nennkapazität der Produktionsanlage,
liegt. Die Zeiträume, in denen die Produktionsanlage unterhalb ihrer Nennkapazität betrieben werden kann, sind nach oben hin nicht begrenzt, so dass eine Produktionsanlage auch längerfristig nach dem erfindungsgemäßen Verfahren unterhalb der Nennkapazität betrieben werden kann, d. h. der Massenstrom des bzw. der eingesetzten Amine m₂ liegt kontinuierlich unterhalb des Massenstroms m₂, _{Soll, Nenn} der eingesetzten Amine bei Nennkapazität der verwendeten Produktionsanlage. Es ist erfindungsgemäß auch möglich, zwischen Betrieb bei Nennkapazität und Betrieb unterhalb der Nennkapazität bei Anwendung der erfindungsgemäß vorgesehenen Maßnahmen hin und her zu wechseln. Das erfindungsgemäße Verfahren erlaubt, eine bestehende Anlage bei unterschiedlichen Auslastungen (unterschiedlichen Produktionskapazitäten) mit gleichbleibender Produkt- und Verfahrensqualität zu betreiben ohne gegenüber der Produktion bei Nennkapazität zusätzliche Kosten für die Aufarbeitung in Kauf zu nehmen. Zusätzlich können beim richtigen Einstellen der Verfahrensparameter unabhängig von der momentanen Produktionskapazität (Nennkapazität, Teillast oder während eines Lastwechsels) auch Ablagerungs- und Anbackungsprobleme im Equipment vermieden werden.

Die vorliegende Erfindung ist sowohl auf die Umsetzung von Aminen **(2)** mit Phosgen **(3)** zu Isocyanaten **(1) in der Flüssig- als auch in der Gasphase** anwendbar.

Wird die Reaktion **in der Flüssigphase** durchgeführt, so wird im stationären Betrieb vor (also im Zeitraum *t_{A}*) oder nach (also im Zeitraum *t_{E}*) einem Lastwechsel bevorzugt ein **Phosgenüberschuss** eingestellt, der einem molaren Verhältnis von Phosgen zu primären Aminogruppen von 1,10: 1 bis 30,0 : 1, besonders bevorzugt von 1,25 : 1 bis 5,00 : 1, entspricht.

Wird die Reaktion in **der Gasphase** durchgeführt, so wird im stationären Betrieb vor (also im Zeitraum *t_{A}*) oder nach (also im Zeitraum *t_{E}*) einem Lastwechsel bevorzugt ein molares Verhältnis von Phosgen zu primären Aminogruppen von 2,50 : 1 bis 5,00 : 1, besonders bevorzugt von 2,60 : 1 bis 4,50 : 1 und ganz besonders bevorzugt von 2,70 : 1 bis 4,00 : 1 eingestellt.

Erfindungsgemäß wird das Verfahren so betrieben, dass der Phosgenüberschuss während der Produktion mit der Produktionskapazität m_{1, Soll} (A) vor Beginn der Übergangsperiode gleich dem Phosgenüberschuss während der Produktion mit der Produktionskapazität m_{1, Soll} (E) nach dem Ende der Übergangsperiode ist.

Die **Übergangsperiode** *t_{Ü}* (also die Zeitspanne des Lastwechsels, die Zeitspanne zwischen den beiden stationären Zuständen) beträgt mindestens 0,50 Minuten, bevorzugt mindestens 5,0 Minuten und besonders bevorzugt mindestens 15 Minuten. Die Übergangsperiode erfährt eine zeitliche Begrenzung im Grunde aus dem Bestreben, die Produktions-Anlage möglichst bald wieder in einem stationären Zustand betreiben zu wollen. Die Übergangsperiode beträgt daher maximal 3 Stunden, bevorzugt maximal 1 Stunde und besonders bevorzugt maximal 30 Minuten, wobei die angegebenen Zeiträume für Mindest- und Maximaldauer der Übergangsperiode beliebig miteinander kombinierbar sind.

Es ist besonders bevorzugt, die Umsetzung im Reaktionsraum **in Gegenwart eines inerten Stoffes (4)** durchzuführen.

Bei der **Flüssigphasenreaktion** handelt es sich bei einem solchen inerten Stoff **(4)** um ein inertes Lösungsmittel. Geeignete inerte *Lösungsmittel (4)* für die Flüssigphasenphosgenierung sind dabei unter den Reaktionsbedingungen inerte Lösungsmittel wie z. B. Monochlorbenzol, Dichlorbenzol (insbesondere das *ortho-Isomer),* Trichlorbenzol, Dioxan, Toluol, Xylol, Methylenchlorid, Perchlorethylen, Trichlorfluormethan, Chlornaphthalin, Decahydronaphthalin oder Butylacetat. Besonders bevorzugt sind Monochlorbenzol und Dichlorbenzol.

Bei der **Gasphasenreaktion** handelt es sich bei einem solchen inerten Stoff **(4)** entweder um ein inertes Gas (d. h. der Stoff (4) liegt bereits bei Raumtemperatur gasförmig vor) oder um die Dämpfe eines inerten Lösungsmittels. Geeignete Inertgase sind z.B. Stickstoff oder Edelgase, wie Helium oder Argon. Besonders bevorzugt ist Stickstoff. Geeignete Lösungsmittel, deren Dämpfe als Inertstoffe (4) Anwendung finden können, sind bspw. Aromaten wie Chlorbenzol, Dichlorbenzol (insbesondere das *ortho*-Isomer), Toluol, Xylol, oder Decahydronaphthalin. Besonders bevorzugt sind Chlorbenzol und Dichlorbenzol.

Im Falle der Verwendung eines inerten Stoffes **(4)** als Verdünnungsmittel ist es zweckmäßig und bevorzugt, das Amin **(2)** dem Reaktionsraum in Form eines Gemisches **(20)** mit dem inerten Stoff **(4)** und/oder das Phosgen **(3)** dem Reaktionsraum in Form eines Gemisches **(30)** mit dem inerten Stoff **(4)** zuzuführen. Dabei können *in den stationären Zuständen* vor und nach dem Lastwechsel die gleichen Konzentrationen an Amin **(2)** im Gemisch **20** und an Phosgen **(3)** im Gemisch **30** gewählt werden, wie im Stand der Technik beschrieben. Bevorzugt werden vor und nach einem Lastwechsel die gleichen Konzentrationen an Amin **(2)** im Gemisch **20** und an Phosgen **(3)** im Gemisch **30** eingestellt.

*Während einer Übergangsperiode* ist es bevorzugt, dass
die durchschnittliche Konzentration des Amins **(2)** im Gemisch **(20)** maximal gleich der durchschnittlichen Konzentration des Amins **(2)** im Gemisch **(20)** während des Zeitraums der Produktion mit der Produktionskapazität m_{1, Soll} (A) ist, und/oder
dass die durchschnittliche Konzentration des Phosgens **(3)** im Gemisch **(30)** während der Übergangsperiode gleich der durchschnittlichen Konzentration des Phosgens **(3)** im Gemisch **(30)** während des Zeitraums der Produktion mit der Produktionskapazität m_{1, Soll} (A) ist.

Die erstgenannte bevorzugte Ausführungsform wirkt der Bildung von Ablagerungen im Reaktionsraum entgegen, da eine ausreichende Verdünnung des Amins sichergestellt wird. Wird die letztgenannte bevorzugte Ausführungsform umgesetzt, so führt die erfindungsgemäße Erhöhung des Phosgenüberschusses während der Übergangsperiode *(Merkmal (iii-2)*) dazu, dass mit dem Phosgen-Inertstoff-Strom **(30)** auch vermehrt inerter Stoff **(4)** in den Reaktionsraum eingetragen wird, was ebenfalls der Bildung von Ablagerungen im Reaktionsraum entgegenwirkt. Der positive Effekt tritt am stärksten auf, wenn beide Ausführungsformen in Verbindung miteinander umgesetzt werden. Wenn im stationären Zustand keine Zugabe eines inerten Stoffes **(4)** vorgesehen ist, so kann der vorstehend beschriebene positive Effekt auch durch Zugabe eines inerten Stoffes **(4)** während der Übergangsperiode *t_{Ü}* direkt in den Reaktionsraum, ohne vorherige Vermischung mit m₂ oder m₃, erfolgen. Bevorzugt erfolgt diese Zugabe eines inerten Stoffes bei *to* oder schon eine Weile, bevorzugt 1,0 Minuten bis 5,0 Minuten, vor *to,* sodass während der kritischen Zeit des Lastwechsels eine ausreichende Verdünnung mit inerten Stoffen gewährleistet ist.

Unabhängig von der Produktionsrate (Nennlast, Teillast oder während eines Lastwechsels) ist eine **ausreichend schnelle Vermischung der Reaktanden** zu gewährleisten. Methoden zur Durchführung kurzer Mischzeiten sind im Prinzip bekannt. In den Mischeinrichtungen können Mischaggregate mit dynamischen oder statischen Mischern eingesetzt werden. In der Flüssigphasen-Phosgenierung werden bevorzugt in den Mischeinheiten dynamische Mischorgane eingesetzt. Als dynamische Mischorgane geeignet sind beispielsweise Mischorgane, wie sie in EP 2 077 150 A1 beschrieben sind. In der Gasphasenphosgenierung werden bevorzugt statische Mischorgane eingesetzt. Als statische Mischorgane geeignet sind beispielsweise Mischorgane, wie sie in EP-A-1 526 129 beschrieben sind.

Geeignete **Isocyanate (1),** die mit dem erfindungsgemäßen Verfahren hergestellt werden können, sind insbesondere aromatische Di- und Polyisocyanate, wie z. B. Methylendiphenyldiisocyanat (MMDI, oder auch Diisocyanate der Diphenylmethanreihe) als Isomere oder als Isomerengemisch, Polymethylenpolyphenylenpolyisocyanat (PMDI, oder auch Polyisocyanate der Diphenylmethanreihe), Gemische aus Methylendiphenyldiisocyanat und Polymethylenpolyphenylenpolyisocyanat (MDI, oder auch Di- und Polyisocyanate der Diphenylmethanreihe), Toluylendiisocyanat (TDI) als reine Isomere oder Isomerengemisch, Isomere des Xylylendiisocyanats (XDI), Isomere des Diisocyanatobenzols, 2,6-Xylolisocyanat, 1,5-Naphthylendiisocyanat (1,5-NDI), Diisocyanate auf Basis von aliphatischen oder cycloaliphatischen Kohlenwasserstoffen mit 2 bis 18 Kohlenstoffatomen, wie z. B. 1,4-Butandiisocyanat, 1,5-Pentandiisocyanat, 1,6-Hexandiisocyanat (HDI), 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat (TMDI), 1,3- und 1,4-Cyclohexandiisocyanat, 1-Isocyanato-3,3,5-trimethyl-5-isocyanatomethylcyclohexan (IPDI), 2,4-, oder 2,6-Diisocyanato-1-methylcyclohexan (H6-TDI), 1-Isocyanato-1-methyl-4(3)-isocyanatomethylcyclohexan (AMCI), 1,3(und/oder 1,4)-Bis(isocyanatomethyl)cyclohexan, Bis(isocyanatomethyl)norbornan (NBDI), 4,4'(und/oder 2,4')-Diisocyanatodicyclohexyl-methan, (cyclo)aliphatische Triisocyanate mit bis zu 22 Kohlenstoffatomen, wie z. B. Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3 -isocyanatopropyl)heptan, 1,6-Diisocyanato-3-(isocyanatomethyl)-hexan und 1,3,5-Tris(isocyanatomethyl)-cyclohexan.

Die korrespondierenden Amine (2) zu den obigen Isocyanaten (1) sind aromatische Di- und Polyamine, wie z. B. Methylendiphenyldiamin (MMDA, oder auch Diamine der Diphenylmethanreihe) als Isomere oder als Isomerengemisch, Polymethylenpolyphenylenpolyamin (PMDA, oder auch Polyamine der Diphenylmethanreihe), Gemische aus Methylendiphenyldiamin und Polymethylenpolyphenylenpolyamin (MDA, oder auch Di- und Polyamine der Diphenylmethanreihe), Toluylendiamin (TDA) als reine Isomere oder Isomerengemisch, Isomere des Xylylendiamins (XDA), Isomere des Diaminobenzols, 2,6-Xylidin, 1,5-Naphthylendiamin (1,5-NDA), Diamine auf Basis von aliphatischen oder cycloaliphatischen Kohlenwasserstoffen mit 2 bis 18 Kohlenstoffatomen, wie z. B. 1,4-Diaminobutan, 1,5-Diaminopentan, 1,6-Diaminohexan (HDA), 1,8-Diaminooctan, 1,9-Diaminononan, 1,10-Diaminodecan, 2,2-Dimethyl-1,5-diaminopentan, 2-Methyl-1,5-pentandiamin (MPDA), 2,4,4(oder -2,2,4)-Trimethyl-1,6-diaminohexan (TMDA), 1,3- und 1,4-Diaminocyclohexan, 1-Amino-3,3,5-trimethyl-5-aminomethylcyclohexan (IPDA), 2,4-, oder 2,6-Diamino-1-methylcyclohexan (H6-TDA), 1-Amino-1-methyl-4(3)-aminomethylcyclohexan (AMCA), 1,3(und/oder 1,4)-Bis(aminomethyl)cyclohexan, Bis(aminomethyl)norbornan (NBDA), 4,4'(und/oder 2,4')-Diaminodicyclohexyl-methan, (cyclo)aliphatische Triamine mit bis zu 22 Kohlenstoffatomen, wie z.B. Triaminocyclohexan, Tris(aminomethyl)-cyclohexan, Triaminomethylcyclohexan, 1,8-Diamino-4-(aminomethyl)octan, 1,6,11-Undecantriamin, 1,7-Diamino-4-(3-aminopropyl)heptan, 1,6-Diamino-3-(aminomethyl)-hexan und 1,3,5-Tris(aminomethyl)-cyclohexan.

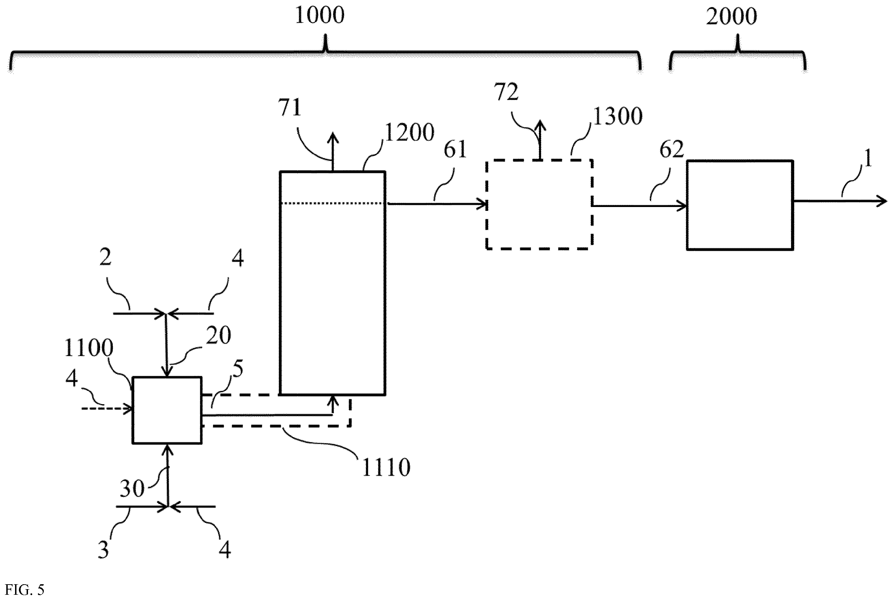

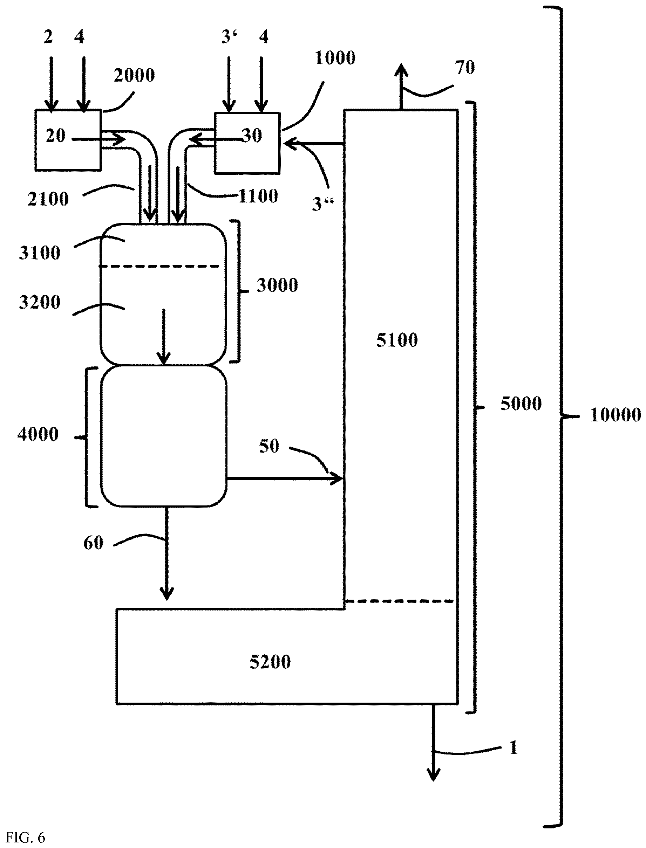

In den stationären Zuständen vor und nach dem Lastwechsel kann das erfindungsgemäße Verfahren betrieben werden wie aus dem Stand der Technik bekannt. **Bevorzugte Ausführungsformen der Flüssig- und Gasphasenphosgenierung im stationären Zustand** werden nachfolgend geschildert:
Das **Verfahren zur Herstellung von Isocyanaten (1) in der Flüssigphase** wird in einer Produktionsanlage umfassend eine Reaktionsstrecke und eine Aufarbeitungsstrecke durchgeführt. Vgl. hierzu auch **FIG. 5****.** (In den folgenden Textabschnitten erhalten die in FIG. 5 gezeigten Apparate das Präfix "5-", um diese von den Apparaten in FIG. 6 (Gasphasenphosgenierung) zu unterscheiden. In der FIG. 5 selbst wird auf dieses Präfix verzichtet.) Dieses Verfahren eignet sich besonders - ohne darauf beschränkt zu sein - für die Herstellung von Di- und Polyisocyanaten der Diphenylmethanreihe und von Toluylendiisocyanat, wobei die Herstellung von Di- und Polyisocyanaten der Diphenylmethanreihe ganz besonders bevorzugt ist. Bevorzugt umfasst das Verfahren folgende Schritte:
I) **Umsetzung** eines Amins (2), bevorzugt in Form einer Aminlösung (20) in einem Lösungsmittel (4), mit Phosgen (3), bevorzugt in Form einer Phosgenlösung (30) in einem Lösungsmittel (4), in der Flüssigphase **in einer Reaktionsstrecke (5-1000)** umfassend
   I.1) eine Mischeinrichtung (5-1100) zur Vermischung von Amin (2) oder Aminlösung (20) mit Phosgen (3) oder Phosgenlösung (30) und optional weiterem Lösungsmittel (4),
   I.2) eine strömungstechnisch nach der Mischeinrichtung angeordnete Reaktionszone (5-1200 - "Phosgenierturm"), wobei zwischen 5-1100 und 5-1200 eine weitere Verweilzeiteinrichtung sein kann (5-1110 - "Suspensionsleitung") und
   I.3) optional, eine Vorrichtung zur Spaltung von Carbaminsäurechlorid (5-1300),
   wobei
   das Amin (2), bevorzugt in Form einer Aminlösung (20), mit einem Massenstrom m₂ und das Phosgen (3), bevorzugt in Form einer Phosgenlösung (30), mit einem Massenstrom m₃ sowie optional Lösungsmittel (4) mit einem Massenstrom m₄ in die Mischeinrichtung 5-1100 geführt und dort vermischt werden, die erhaltene Mischung in der nachgeschalteten Reaktionszone 5-1200 weiter umgesetzt wird und in einen flüssigen, Roh-Isocyanat und Lösungsmittel (sowie Spuren an Phosgen und Chlorwasserstoff) enthaltenden Strom (61, 62) und einen gasförmigen, Phosgen und Chlorwasserstoff (sowie Spuren an Lösungsmittel) enthaltenden Strom (71, 72) aufgetrennt wird;
   II) bis VI), bevorzugt II) bis XII), **Aufarbeitung des Roh-Isocyanats in einer Aufarbeitungsstrecke** (*5*-**2000) zu dem gewünschten Isocyanat-Endprodukt (1),** wobei die Schritte im Einzelnen Folgendes umfassen:
II) Trennung des flüssigen Stroms (61 bzw. 62) aus Schritt I) in einen flüssigen, Lösungsmittel und Roh-Isocyanat (sowie Spuren an Phosgen) enthaltenden Strom (80) und einen gasförmigen, Phosgen und Chlorwasserstoff (sowie Spuren an Lösungsmittel) enthaltenden Strom (90) in einer Destillationsvorrichtung (5-2100 - "Entphosgenierkolonne");
III) Trennung des flüssigen Stroms (80) in einen gasförmigen, Lösungsmittel (sowie Spuren an Phosgen) enthaltenden Strom (110) und eine flüssigen, Roh-Isocyanat (sowie Spuren an Lösungsmittel) enthaltenden Strom (100) in einer Destillationsvorrichtung (5-2200 - "Lösungsmittelkolonne");
IV) Trennung des gasförmigen Stroms (110), bevorzugt nach dessen Verflüssigung in einem Kondensator (5-2310), in einen flüssigen, Lösungsmittel enthaltenden Strom (120) und einen gasförmigen, Phosgen enthaltenden Strom (130) in einer Destillationsvorrichtung (5-2300 - "Lösungsmittelstripper");
V) Gewinnung eines flüssigen Isocyanat-Stroms (140) aus dem flüssigen Strom (100), wobei ein Nebenkomponenten und ggf. Lösungsmittel enthaltender gasförmiger Strom (150) anfällt, in einer Destillationsvorrichtung (5-2400), bevorzugt umfassend die Abtrennung von polymeren Isocyanatfraktionen (141) in einer vorgeschalteten Einrichtung zur Polymerabtrennung (5-2410 - "Polymerabtrennung", PMA);
VI) Absorption der gasförmigen Ströme (71), (72), (90) und (130) in Lösungsmittel (4) unter Erhalt eines flüssigen, Lösungsmittel und Phosgen enthaltenden Stroms (160) und eines gasförmigen, Chlorwasserstoff enthaltenden Stroms (170) in einer Absorptionsvorrichtung (5-2500 - "Phosgenabsorber");
VII) optional und bevorzugt, Absorption des gasförmigen Stroms (170) in Wasser oder verdünnter Salzsäure in einer weiteren Absorptionsvorrichtung (5-2600 - "HCl-Absorptionskolonne");
VIII) optional und bevorzugt, Reinigung von Abgasströmen mindestens aus VII), bevorzugt Reinigung von Abgasströmen aus allen vorhandenen Anlagenteilen, in einer Vorrichtung zur Abgasreinigung (5-3000).

Die Herstellung von Isocyanaten (1) **in einem stationären Zustand** nach diesem Verfahren lässt sich exemplarisch wie folgt zusammenfassen (vgl. hierzu auch FIG. 5; die Aufarbeitungsstrecke 5-2000 ist dort nur rein schematisch gezeigt ohne auf Details einzugehen):
a) Kemvorgang des Schritts I): das Amin wird mit Phosgen in einem Lösungsmittel zum entsprechenden Isocyanat umgesetzt. Dies geschieht in einem Reaktionsraum mindestens umfassend die Mischeinrichtung 5-1100 und die Reaktionszone 5-1200. Das erhaltene rohe Verfahrensprodukt wird in einen flüssigen, das Roh-Isocyanat, ggf. teilweise in Form des entsprechenden Carbaminsäurechlorids, und Lösungsmittel (sowie Spuren an Phosgen und Chlorwasserstoff) enthaltenden Strom (61) und einen gasförmigen, Phosgen und Chlorwasserstoff (sowie Spuren an Lösungsmittel) enthaltenden Strom (71) aufgetrennt. Ist die Vorrichtung 5-1300 vorhanden, so fallen dort ein flüssiger Strom 62 (enthaltend das Roh-Isocyanat und Lösungsmittel (sowie Spuren an Phosgen und Chlorwasserstoff)) und ein gasförmiger Strom 72 (enthaltend überwiegend Chlorwasserstoff sowie Spuren an Phosgen) an.
b) Kernvorgang des Schrittes II): Abtrennung weiteren bei der Reaktion entstandenen Chlorwasserstoffes zusammen mit nicht umgesetztem Phosgen aus dem Strom 61 (falls 5-1300 vorhanden ist: aus dem Strom 62) in der sog. Entphosgenierkolonne *5*-2100 (nicht gezeigt in FIG. 5).
c) Kernvorgang des Schrittes III): Abtrennung von Lösungsmittel aus dem in Schritt II) erhaltenen flüssigen Strom 80 in der sog. Lösungsmittelkolonne *5*-2200 (nicht gezeigt in FIG. 5).
d) Kernvorgang des Schrittes IV): Abtrennung von Phosgen aus dem in Schritt III) erhaltenen gasförmigen Strom 110, bevorzugt nach dessen Verflüssigung in einem Kondensator (*5*-2310), im sog. Lösungsmittelstripper *5*-2300 (nicht gezeigt in FIG. 5).
e) Kernvorgang des Schrittes V): Abtrennung von Lösungsmittel aus dem im Schritt III) erhaltenen flüssigen Strom 100 mittels Destillation (*5*-2400), bevorzugt umfassend die Abtrennung polymerer Isocyanatfraktionen (141), unter Erhalt des Isocyanatstroms 140 (nicht gezeigt in FIG. 5).
f) Kernvorgang des Schrittes VI): Absorption der gasförmigen Ströme aus den Schritten I), II) und III) in einem Lösungsmittel im sog. Phosgenabsorber *5*-2500 (nicht gezeigt in FIG. 5).
g) Kernvorgang des Schrittes VII) (optional): Absorption des in Schritt VI) erhaltenen Gasstroms 170 in Wasser oder verdünnter Salzsäure in der sog. HCl-Absorptionskolonne *5*-2600 (nicht gezeigt in FIG. 5).
h) Kernvorgang des Schrittes VIII) (optional): Reinigung der Abgasströme aus Schritt VII), bevorzugt der Abgasströme aus allen Schritten, in einer Abgasreinigungsvorrichtung *5*-3000 (nicht gezeigt in FIG. 5).

Die kontinuierliche oder semi-kontinuierliche, bevorzugt kontinuierliche, Produktion des Isocyanats **in a)** erfolgt in einer Reaktionsstrecke nach einem aus dem Stand der Technik bekannten Verfahren. Geeignete Verfahren sind beispielsweise in EP 2 077 150 B1, EP 1 616 857 A1, EP 1 873 142 A1, EP 0 716 079 B1 oder EP 0 314 985 B1 beschrieben. Konzentrationen und Mengenströme der Edukte Amin und Phosgen werden dabei jedoch bevorzugt so gewählt, dass sich in der Mischzone ein molares Verhältnis von Phosgen zu primären Aminogruppen von 1,10 : 1 bis 30,0 : 1, besonders bevorzugt von 1,25 : 1 bis 5,00 : 1, einstellt. Alle Verfahren für die Produktion eines Isocyanats in der Flüssigphase liefern ein rohes Verfahrensprodukt, das in eine Flüssigphase (61, 62), enthaltend neben dem gewünschten Isoyanat gelösten Chlorwasserstoff, überschüssiges gelöstes Phosgen und Lösungsmittel, und eine Gasphase (71, 72), enthaltend Chlorwasserstoffgas, überschüssiges gasförmiges Phosgen und gasförmiges Lösungsmittel, zerfällt bzw. aufgetrennt wird.

Die weitere Abtrennung von Chlorwasserstoff und Phosgen aus dem flüssigen Roh-Isocyanat-Strom 61 bzw. 62 in der sog. Entphosgenierkolonne *5*-2100 **in b)** kann nach einem beliebigen aus dem Stand der Technik bekannten Verfahren erfolgen, bevorzugt wie in DE-A-10260084 beschrieben.

Die weitere Abtrennung von Lösungsmittel aus so erhaltenem flüssigen Isocyanat-Strom 80 in der sog. Lösungsmittelkolonne *5*-2200 **in c)** kann nach einem beliebigen aus dem Stand der Technik bekannten Verfahren erfolgen, bevorzugt wie in EP 1 854 783 B1 beschrieben.

Die Abtrennung von Phosgen aus dem so erhaltenen gasförmigen Lösungsmittel-Strom 110, bevorzugt nach dessen Verflüssigung in einem Kondensator (*5*-2310), im sog. Lösungsmittelstripper *5*-2300 **in d)** kann nach einem beliebigen aus dem Stand der Technik bekannten Verfahren erfolgen, bevorzugt wie in EP 1 854 783 B1 beschrieben.

Die Abtrennung von Lösungsmittel aus dem im Schritt III) erhaltenen flüssigen Isocyanat-Strom 100 **in e),** ggf. umfassend eine Polymerabtrennung, kann nach einem beliebigen aus dem Stand der Technik bekannten Verfahren erfolgen. Geeignete Verfahren sind beschrieben in EP 1 854 783 A2 und EP 1 506 957 A1, oder auch in EP 1 371 635 B1.

Die Absorption der gasförmigen Ströme aus den Schritten I), II) und III) in einem Lösungsmittel im sog. Phosgenabsorber *5*-2500 **in f)** kann nach einem beliebigen aus dem Stand der Technik bekannten Verfahren erfolgen, bevorzugt wie in DE-A-10260084 oder EP 2 093 215 A1 beschrieben.

Die Absorption des so erhaltenen HCl-Gasstroms 170 in Wasser oder verdünnter Salzsäure in der sog. HCl-Absorptionskolonne 5-2600 zur Gewinnung von Salzsäure **in g)** kann nach einem beliebigen aus dem Stand der Technik bekannten Verfahren erfolgen. Bevorzugt sind Verfahrensweisen, wie in EP 2 021 275 B1 und EP 1 743 882 B1 beschrieben.

Der Verfahrensschritt der Prozessabgasbehandlung **in h)** kann gemäß einem aus dem Stand der Technik bekannten Verfahren erfolgen.

Das **Verfahren zur Herstellung von Isocyanaten (1) in der Gasphase** wird in einer Produktionsanlage (10000) durchgeführt (vgl. **FIG. 6****).** (In den folgenden Textabschnitten erhalten die in FIG. 6 gezeigten Apparate das Präfix "*6*-", um diese von den Apparaten in FIG. 5 (Flüssigphasenphosgenierung) zu unterscheiden. In der FIG. 6 selbst wird auf dieses Präfix verzichtet.) Dieses Verfahren eignet sich ganz besonders für die Herstellung von Toluylendiisocyanat, ist jedoch nicht darauf beschränkt. Die Gasphasenphosgenierungsanlage (*6*-1000) enthält mindestens
(i) eine Vorrichtung 6-**1000** zur Bereitstellung eines gasförmigen Phosgenstroms, optional umfassend neben Phosgen (**3**) einen Inertstoff (**4**),
(ii) eine Vorrichtung 6-**2000** zur Bereitstellung eines gasförmigen Aminstroms, optional umfassend neben Amin (**2**) einen Inertstoff (**4**),
(iii) eine Mischzone (6-**3100**) zur Vermischung der Ströme gasförmigen Amins und gasförmigen Phosgens, wobei die Mischzone durch Einrichtungen (6-**1100**, 6-**2100**) jeweils mit der Vorrichtung 6-**1000** und der Vorrichtung 6-**2000** verbunden ist,
(iv) eine strömungstechnisch nach der Mischzone (6-**3100**) angeordnete Reaktionszone (6-**3200**) zur weiteren Umsetzung der zuvor vermischten Ströme gasförmigen Amins und gasförmigen Phosgens,
(v) eine strömungstechnisch nach der Reaktionszone (6-**3200**) angeordnete Reaktionsabbruchzone (6-**4000**) zur Beendigung der Umsetzung,
   und optional
(vi) einen Aufarbeitungsteil (6-**5000**) umfassend Einrichtungen zur Rückgewinnung und Rückführung nicht umgesetzten Phosgens (**3"**) (*6*-**5100**) und Einrichtungen zur Gewinnung des hergestellten Isocyanats (**1**) in Reinform (6-**5200**)

(i) Als *Vorrichtung zur Bereitstellung eines gasförmigen Phosgenstroms (6-**1000**)* kann grundsätzlich jede aus dem Stand der Technik bekannte, für die Überführung von Phosgen in die Gasphase geeignete, Vorrichtung verwendet werden. Bevorzugt erfolgt die Phosgengaserzeugung durch Destillation oder partielle Verdampfung in einer Destillationskolonne, wie in DE 10 2009 032413 A1 in den Abschnitten [0081] bis [0118] beschrieben. Die Energiezufuhr im Sumpf der Kolonne kann durch jeden denkbaren Verdampfer erfolgen, wie zum Beispiel Naturumlaufverdampfer, Kletterverdampfer und Fallfilmverdampfer. Insbesondere bevorzugt sind Fallfilmverdampfer. In der Vorrichtung 6-1000 wird das in der Phosgenierung verwendete Phosgen (**3**) auf eine Temperatur von 200 °C bis 600 °C, bevorzugt von 250 °C bis 420 °C, besonders bevorzugt von 250 °C bis 400 °C, erhitzt.
(ii) Als *Vorrichtung zur Bereitstellung eines gasförmigen Aminstroms (6-**2000**)* kann grundsätzlich jede aus dem Stand der Technik bekannte für die Überführung eines Amins in die Gasphase geeignete Vorrichtung, wie dem Fachmann bekannte Verdampfungsapparaturen, verwendet werden. In einer bevorzugten Ausführungsform umfasst die Vorrichtung *6*-**2000** eine Einrichtung zur Verdampfung und eine Einrichtung zur anschließenden Überhitzung des Amins (**2**). Ganz besonders bevorzugt sind mehrstufige Verdampfungs- und Überhitzungssysteme, in denen zwischen den Verdampfungs- und Überhitzungssystemen Tröpfchenabscheider eingebaut sind und/oder die Verdampfungsapparaturen auch die Funktion eines Tröpfchenabscheiders besitzen. Geeignete Tröpfchenabscheider sind z. B. beschrieben in "Droplet Separation", A. Bürkholz, VCH Verlagsgesellschaft, Weinheim - New York - Basel - Cambridge, 1989. Nach Verlassen des in Strömungsrichtung letzten Überhitzers wird der auf seine Solltemperatur vorgeheizte gasförmige Aminstrom (**20** in FIG. 6) dem Reaktionsraum zugeführt. In der Vorrichtung 6-2000 wird das Amin (2) verdampft und auf eine Temperatur von 200 °C bis 600 °C, bevorzugt von 250 °C bis 420 °C, besonders bevorzugt von 250 °C bis 400 °C, erhitzt.
(iii) Der Aufbau einer einsetzbaren *Mischzone (6-3100)* kann auf dem Fachmann bekannte Art und Weise erfolgen, bevorzugt wie in EP-A-2 196 455, insbesondere in den Absätzen [0047] bis [0049], und EP-A-1 935 876, insbesondere in den Absätzen [0027] bis [0029], beschrieben. Die Mischzone beginnt dort, wo im Regelbetrieb die Ströme gasförmigen Amins und gasförmigen Phosgens (**20** bzw. **30** in FIG. 6) erstmals aufeinandertreffen.
(iv) Die in der Mischzone (6-**3100)** erstmals aufeinandertreffenden Amin- und Phosgengasströme werden in einem Verweilzeitapparat, der *Reaktionszone (6-**3200**),* weiter umgesetzt. Mischzone (6-**3100**) und Reaktionszone (6-**3200**) können bevorzugt auch in einem einzigen Apparat, dem *Reaktor (6-**3000**)* vereinigt sein wie in EP 2 196 455 A1, insbesondere in den Absätzen [0042] bis [0049], beschrieben.

Die *Einrichtungen 6-**1100** und 6-**2100**,* welche die Vorrichtungen zur Bereitstellung des gasförmigen Phosgen- (*6*-**1000**) bzw. Amingasstroms (*6*-**2000**) mit der Mischzone (6-**3100**) verbinden, sind solche Einrichtungen, welche sich zur Überführung des jeweiligen Gasstroms aus den Vorrichtungen *6*-**1000** bzw. *6*-**2000** in die Mischzone (*6*-**3100**) eignen. Diese Einrichtungen umfassen neben Rohrleitungen zum Transport der Gasströme bevorzugt auch Düsenvorrichtungen, die eine intensive Durchmischung von Phosgengasstrom (**30** in FIG. 6) und Amingasstrom (**20** in FIG. 6) in der Mischzone (*6*-**3100**) gewährleisten. Es ist möglich, den Amin- und Phosgengasstrom einzeln in die Mischzone (*6*-**3100**) einzudüsen. Bevorzugt ist jedoch eine Ausführungsform, in welcher die Rohrleitungen der Einrichtungen 6-**1100** und 6-**2100** in eine gemeinsame Düsenvorrichtung einmünden (in FIG. 1 nicht gezeigt). In dieser Ausführungsform wird einer der beiden Gasströme, bevorzugt der Amingasstrom, der Mischzone **(6-3100)** über eine zentral in einem bevorzugt zylindrischen Behälter angeordnete Innendüse zugeführt. Der andere Gasstrom, bevorzugt der Phosgengasstrom, wird über den durch die Außenwand der Innendüse und der Innenwand des Behälters gebildeten Ringraum eingetragen. An der Austrittsöffnung der Innendüse (= Beginn der Mischzone) vermischen sich die beiden Gasströme. Eine solche Ausführungsform ist beispielsweise in FIG. 1 von EP-A-1 449 826 und in FIG. 1 von EP-A-1 362 847 dargestellt. In diesem Fall sind die Einrichtungen 6-**1100** und 6-**2100** teilweise in einander und in die Mischzone (6-**3100**) integriert. Es ist auch möglich, wie in FIG. 2 von EP-A-1 449 826 dargestellt, an Stelle einer einzigen Zentraldüse eine Anordnung aus mehreren Einzeldüsen zu verwenden. Weitere einsetzbare Ausführungsformen für die Einrichtungen *6*-**1100** und *6*-**2100** sind beispielsweise in EP-A-2 196 455, insbesondere in den Absätzen [0047] bis [0048], und EP-A-1 935 876, insbesondere in den Absätzen [0027] und [0028], beschrieben.

(v) Einsetzbare *Reaktionsabbruchzonen (6-**4000**)* sind dem Fachmann bekannt. Bevorzugt ist eine Ausführungsform, wie sie in EP 1 935 875 B1, insbesondere in den Absätzen [0024] und [0025], beschrieben ist. In der Reaktionsabbruchzone **(***6***-4000)** wird das gasförmige Rohprodukt der Reaktion, welches neben dem Isocyanat (1) im Wesentlichen noch das Koppelprodukt Chlorwasserstoff und nicht umgesetztes Phosgen enthält, rasch abgekühlt, bevorzugt durch Eindüsen eines inerten Lösungsmittels (bevorzugt *ortho*-Dichlorbenzol, ODB), ggf. gemeinsam mit einem Teil des zuvor gebildeten und rezyklierten Isocyanats (**1**), in den Rohprodukt-Gasstrom. Bevorzugt wird das rohe Reaktionsprodukt in der Reaktionsabbruchzone (6-**4000**) in einen gasförmigen Anteil (Brüden, **50**) und einen flüssigen Anteil (**60**) getrennt.

In einer besonders bevorzugten Ausgestaltung des Verfahrens wird das in der Reaktionsabbruchzone (*6*-**4000**) erhaltene Rohprodukt in derselben Gasphasenphosgenierungsanlage (*6*-**10000**) aufgearbeitet, um das Isocyanat (**1**) aus dem flüssigen Gemisch (**60**) zu isolieren. In diesem Fall umfasst die Gasphasenphosgenierungsanlage (*6*-**10000**) zusätzlich
(vi) einen *Aufarbeitungsteil (6-**5000**).*

Geeignete Vorrichtungen zur Aufarbeitung sind in WO 2011/003532, insbesondere Seite 5, Zeile 19 bis Seite 28, Zeile 5, und in EP 1 371 636 B1, EP 1 371 635 B1 und EP 1 413 571 B1, jeweils das gesamte Dokument, beschrieben. Der Aufarbeitungsteil (*6*-**5000**) lässt sich unterteilen in *Einrichtungen zur Rückgewinnung und Rückführung nicht umgesetzten Phosgens (sowie zur Abtrennung des Koppelprodukts Chlorwasserstoff) (6-**5100**)* und in *Einrichtungen zur Gewinnung des hergestellten Isocyanats in Reinform (sowie ggf. zur Rückgewinnung inerten Lösungsmittels) (6-**5200**).* Der Aufarbeitungsteil ist in FIG. 6 nur schematisch angedeutet ohne die nachfolgend aufgeführten Details. Insbesondere bevorzugt umfasst der Aufarbeitungsteil (*6*-**5000**) eine *Auswaschkolonne (6-**5110**)* zur Entfernung von Isocyanat aus den Brüden (**50**) der Reaktionsabbruchszone (*6*-**4000**) durch Waschen mit einem inertem Lösungsmittel, eine *Phosgenabsorptionskolonne (6-**5120**)* zur Wiedergewinnung von Phosgen aus den Brüden der Auswaschkolonne (*6*-**5110**) durch Absorption in einem inerten Lösungsmittel, wodurch Chlorwasserstoff und Inerte (**70**) vom Phosgen getrennt werden, eine *Phosgendesorptionskolonne (6-**5130**)* zur Trennung von Phosgen und inertem Lösungsmittel, eine *Lösungsmittelkolonne (6-**5210**)* insbesondere zur Abtrennung von Leichtsiedern (insbesondere inertem Lösungsmittel aus der Reaktionsabbruchzone) aus dem rohen Isocyanat, eine *Feinreinigungskolonne (6-**5220**)* insbesondere zur Abtrennung von Schwersiedern (z. B. Polyharnstoff-haltigen Rückständen) aus dem in der Lösungsmittelkolonne vorgereinigten Isocyanat, so dass gereinigtes Endprodukt erhalten wird.

Das für beide Verfahrensführungen, Flüssig- und Gasphasenphosgenierung, erforderliche **Phosgen (3)** kann grundsätzlich nach allen aus dem Stand der Technik bekannten Verfahren hergestellt werden. Hierfür kann ein "Kaltvereiniger" entsprechend EP 1 640 341 B1 oder ein "Heißvereiniger" entsprechend EP 0 134 506 B1 benutzt werden. Bei der sogenannten - bevorzugt eingesetzten - Heißvereinigung (siehe EP 0 134 506 B1) wird Phosgen durch Umsetzung von Chlor mit Kohlenmonoxid in Aktivkohle als Katalysator enthaltenen Rohrreaktoren unter gleichzeitiger Nutzung der anfallenden Reaktionswärme zur Erzeugung von Dampf umgesetzt. Dabei wird in einem ersten, gekörnte Aktivkohle enthaltenen Rohrreaktor mit einem lichten Rohrdurchmesser von maximal 100 mm 95 Vol.-% bis 98 Vol.-% des eingesetzten Chlors mit überschüssigem Kohlenmonoxid bei Reaktionstemperaturen von über 250 °C zu Phosgen umgesetzt. Die hierbei anfallende Reaktionswärme wird durch Verdampfungskühlung einer bei 150 °C bis 320 °C siedenden Flüssigkeit oder mit einer nicht-siedenden Flüssigkeit abgeführt, deren Temperatur am Reaktoraustritt mittels Zwangsumlaufpumpen und Temperatursteuerung bei 150 °C bis 320 °C gehalten wird. Der den Reaktor verlassende flüssige oder dampfförmige Wärmeträger wird in einem mit Wasser als Kühlmedium beschickten Wärmetauscher unter Erzeugung von Dampf kondensiert und / oder auf eine unter der Temperatur des Wärmeträgers am Reaktoraustritt liegende Temperatur abgekühlt und in den Reaktor zurückgeführt. Die den Reaktor verlassenden Reaktionsgase werden auf eine Temperatur von 50 °C bis 120 °C abgekühlt und anschließend in einen gekörnte Aktivkohle enthaltenden zweiten Reaktor geleitet, dessen Temperatur auf 50 °C bis 100 °C thermostatisch eingestellt und in dem die Umsetzung zu Ende geführt wird, so dass das den zweiten Reaktor verlassende Phosgen einen Restchlorgehalt von weniger als 50 ppmv aufweist. Das am Kopf des Reaktors austretende Phosgen wird, wie oben beschrieben, kondensiert.

Durch die erfindungsgemäße Vorgehensweise ergeben sich die folgenden Vorteile für die Herstellung von Isocyanaten (1):
i) Die Produktivität der Phosgenier-Anlage ist höher, weil weniger Reinigungszeiten insbesondere für die Reaktionsstrecke und die nachfolgenden Anlagensegmente nötig sind.
ii) Die Produktivität der Phosgenier-Anlage ist höher, weil weniger Druckverluste in den Mischaggregaten und Rohrleitungen der Reaktionsstrecke auftreten.
iii) Die Energieeffizienz der Reaktionsstrecke ist höher, weil weniger Ablagerungen an den Apparatewänden eine bessere Wärmeübertragung gewährleisten.
iv) Es entsteht weniger Abfall nach der Reinigung der Reaktionsstrecke und nachfolgender Anlagensegmente (Polyharnstoffbildung minimiert).
v) Die Bildung von Feststoffen, die die nachgeschalteten Apparate wie Pumpen und Kolonnen durch Abrasion oder Ablagerungen beeinträchtigen können, wird minimiert.
vi) Niedriger spezifischer und gegebenenfalls absoluter Phosgen-Hold-Up.
vii) Einsparung von Energien für die Aufarbeitung des Lösungsmittels in der Flüssigphase.
viii) Einsparung von Energien für die Rezyklierung von Phosgen.
ix) Reduktion der thermischen Belastung des Roh-Isocyanates.

Somit ermöglicht die erfindungsgemäße Verfahrensweise während eines nicht-stationären Zustandes (während einer Übergangsperiode) einen technisch reibungslosen Lastwechsel ohne anschließende Ausfallzeiten im darauffolgenden stationären Zustand mit gleichbleibend hoher Qualität des angestrebten Isocyanat-Endprodukts. Das erfindungsgemäße Verfahren ermöglicht auch einen zügigen Lastwechsel und somit ein schnelles Reagieren auf Ereignisse wie Rohstoffmangel etc.

Die vorliegende Erfindung wird anhand der nachfolgenden Zeichnungen und Beispiele noch weiter erläutert, ohne jedoch darauf beschränkt zu sein.

Es zeigen:
- **FIG. 1-4**: den zeitlichen Verlauf der Massenströme von Phosgen (3) und Amin (2) während der Übergangsperiode.

Die Abbildungen zeigen jeweils den zeitlichen Verlauf der Massenströme von Amin und Phosgen in verschiedenen Ausführungsformen im Hinblick auf die Ausgestaltung der Übergangsperiode derart, dass die erfindungsgemäßen Merkmale (iii-1) und (iii-2) erfüllt sind (im Hinblick auf das Massenverhältnis von Phosgen zu Amin nach dem Zeitpunkt ti im Vergleich zum Massenverhältnis von Phosgen zu Amin vor dem Zeitpunkt t₀ spiegeln die Abbildungen das erfindungsgemäße Verfahren nicht quantitativ wider). Auf der x-Achse ist jeweils die Zeit *t,* wobei die Zeit bis *t₀* und nach *t₁* die jeweils stationären Anlagenzustände beschreibt, und auf der y-Achse sind jeweils die Massenströme m₂ und m₃ aufgetragen. Bis zum Erreichen des Zeitpunkts *to* sind die Massenströme für Amin (m_{2,} in der Figur mit "A" bezeichnet) und für Phosgen (m₃, in der Figur mit "P" bezeichnet) in den in der Terminologie der vorliegenden Erfindung als Reaktionsraum bezeichneten Phosgenier-Reaktor konstant.

**FIG. 1**: Es wird beschlossen, dass die Produktionskapazität **reduziert** werden soll. Hierzu wird bei unveränderter Höhe des Massenstroms an Phosgen beginnend beim Zeitpunkt *to* der Massenstrom an Amin in den Reaktionsraum reduziert, bis er zum Zeitpunkt *t₁* den angestrebten Zielwert m_{2,Soll}(E) erreicht.

Zum Zeitpunkt *t₂* wird begonnen, den Massenstrom des Phosgens in den Reaktionsraum zu reduzieren, bis er zum Zeitpunkt *t₁* ebenfalls den angestrebten Zielwert (m₃, _{Soll} (E)) erreicht. Man erkennt, dass der momentane Phosgenüberschuss bei *t₂* größer ist als vor *to* (Merkmal (iii-1)) und dass der durchschnittliche Phosgenüberschuss während der Übergangsperiode größer ist als der Phosgenüberschuss vor *to* (Merkmal (iii-2)).

Nach der Zeit *t₁*, wenn der angestrebte stationäre Zustand erreicht ist, sind die Massenströme für Amin (m_{2,} in der Figur mit "A" bezeichnet) und für Phosgen (m₃, in der Figur mit "P" bezeichnet) in den in der Terminologie der vorliegenden Erfindung als Rektionsraum bezeichneten Phosgenier-Reaktor wieder konstant, und das Massenverhältnis von Phosgen zu Amin ist - in dieser Hinsicht weicht FIG. 1 von der Erfindung wie in Patentanspruch 1 definiert ab - kleiner als vor dem Zeitpunkt *to* (natürlich unter Beachtung des Merkmals (ii)). Die Massenströme von Phosgen und Amin erreichen zeitgleich den angestrebten stationären Zustand.

Im nicht-stationären Zustand zwischen *to* und *t₁* kann die Änderung der Zugabe von Phosgen und Amin nicht nur linear verlaufen, sondern auch nicht-linear mit einem kurvenförmigen Verlaufsprofil, sofern die sonstigen Erfordernisse der Erfindung eingehalten werden.

FIG. 2: Es wird beschlossen, dass die Produktionskapazität **reduziert** werden soll. Hierzu wird bei unveränderter Höhe des Massenstroms an Phosgen zum Zeitpunkt *t*₀ der Massenstrom an Amin in den Reaktionsraum reduziert, bis er zum Zeitpunkt t₃ den angestrebten Zielwert erreicht m_{2, Soll} (E).

Zum Zeitpunkt *t₂* wird begonnen, den Massenstrom des Phosgens in den Reaktionsraum zu reduzieren, bis er zum Zeitpunkt *t₁* ebenfalls den angestrebten Zielwert (m₃, _{Soll} (E)) erreicht. Man erkennt, dass der momentane Phosgenüberschuss bei *t₂* größer ist als vor *to* (Merkmal (iii-1)) und dass der durchschnittliche Phosgenüberschuss während der Übergangsperiode größer ist als der Phosgenüberschuss vor *to* (Merkmal (iii-2)).

Nach der Zeit *t₁*, wenn der angestrebte stationäre Zustand erreicht ist, sind die Massenströme für Amin (m_{2,} in der Figur mit "A" bezeichnet) und für Phosgen (m₃, in der Figur mit "P" bezeichnet) in den in der Terminologie der vorliegenden Erfindung als Reaktionsraum bezeichneten Phosgenier-Reaktor wieder konstant. Die Massenströme von Phosgen und Amin erreichen nicht zeitgleich den angestrebten stationären Zustand. Der Massenstrom von Amin ist zu einem früheren Zeitpunkt (*t₃*) wieder konstant als der von Phosgen, so dass zum Zeitpunkt *t₃* zwar schon die angestrebte Produktionskapazität erreicht ist, aber zwischen *t₃* und *t₂* der Phosgenmassenstrom m₃ weiter reduziert wird, bis schließlich bei t*₁* beide Massenströme im stationären Zustand sind.

Im nicht-stationären Zustand zwischen t₀ und t₁ kann die Änderung der Zugabe von Phosgen und Amin nicht nur linear verlaufen, sondern auch nicht-linear mit einem kurvenförmigen Verlaufsprofil, sofern die sonstigen Erfordernisse der Erfindung eingehalten werden.

**FIG. 3****:** Es wird beschlossen, dass die Produktionskapazität **erhöht** werden soll. Hierzu wird bei unveränderter Höhe des Massenstroms an Amin zum Zeitpunkt *to* der Massenstrom an Phosgen in den Reaktionsraum erhöht, bis er zum Zeitpunkt *t₁* den angestrebten Zielwert (m₃, _{Soll} (E)) erreicht.

Zum Zeitpunkt *t₂* wird begonnen, den Massenstrom des Amins in den Reaktionsraum zu erhöhen, bis er zum Zeitpunkt *t₁* ebenfalls den angestrebten Zielwert (m_{2, Soll} (E)) erreicht. Man erkennt, dass der momentane Phosgenüberschuss bei *t₂* größer ist als vor *to* (Merkmal (iii-1)) und dass der durchschnittliche Phosgenüberschuss während der Übergangsperiode größer ist als der Phosgenüberschuss vor *to* (Merkmal (iii-2)).

Nach der Zeit *t₁*, wenn der angestrebte stationäre Zustand erreicht ist, sind die Massenströme für Amin (m_{2,} in der Figur mit "A" bezeichnet) und für Phosgen (m₃, in der Figur mit "P" bezeichnet) in den in der Terminologie der vorliegenden Erfindung als Reaktionsraum bezeichneten Phosgenier-Reaktor wieder konstant. Die Massenströme von Phosgen und Amin erreichen zeitgleich den angestrebten stationären Zustand und damit die gewünschte Produktionskapazität.

Im nicht-stationären Zustand zwischen *to* und *t₁* kann die Änderung der Zugabe von Phosgen und Amin nicht nur linear verlaufen, sondern auch nicht-linear mit einem kurvenförmigen Verlaufsprofil, sofern die sonstigen Erfordernisse der Erfindung eingehalten werden.

**FIG. 4****:** Es wird beschlossen, dass die Produktionskapazität **erhöht** werden soll. Hierzu wird bei unveränderter Höhe des Massenstroms an Amin zum Zeitpunkt *to* der Massenstrom an Phosgen in den Phosgenier-Reaktor erhöht, bis er zum Zeitpunkt *t₃* den angestrebten Zielwert (m₃, _{Soll} (E)) erreicht.

Zum Zeitpunkt *t₂* wird begonnen, den Massenstrom des Amins in den Reaktionsraum zu erhöhen, bis er zum Zeitpunkt *t₁* ebenfalls den angestrebten Zielwert (m_{2, Soll} (E)) erreicht. Man erkennt, dass der momentane Phosgenüberschuss bei *t₂* größer ist als vor *to* (Merkmal (iii-1)) und dass der durchschnittliche Phosgenüberschuss während der Übergangsperiode größer ist als der Phosgenüberschuss vor *to* (Merkmal (iii-2)).

Nach der Zeit *t₁,* wenn der angestrebte stationäre Zustand erreicht ist, sind die Massenströme für Amin (m_{2,} in der Figur mit "A" bezeichnet) und für Phosgen (m₃, in der Figur mit "P" bezeichnet) in den in der Terminologie der vorliegenden Erfindung als Reaktionsraum bezeichneten Phosgenier-Reaktor wieder konstant. Die Massenströme von Phosgen und Amin erreichen nicht zeitgleich den angestrebten stationären Zustand. Der Massenstrom von Phosgen ist zu einem früheren Zeitpunkt (*t₃*) als der von Amin wieder konstant, so dass zum Zeitpunkt *t₃* noch nicht die angestrebte Produktionskapazität erreicht ist. In der Zeit zwischen *t₃* und *t₁* wird weiterhin der Aminmassenstrom erhöht bis bei *t₁* beide Massenströme im stationären Zustand sind. Erst jetzt ist die angestrebte Produktionskapazität erreicht.

Im nicht-stationären Zustand zwischen *to* und *t₁* kann die Änderung der Zugabe von Phosgen und Amin nicht nur linear verlaufen, sofern die sonstigen Erfordernisse der Erfindung eingehalten werden.

### Beispiele

### Allgemeine Bedingungen für die Herstellung eines Gemisches aus Methylendiphenyldiisoyanat und Polymethylenpolyphenylenpolyisocyanat (nachfolgend summarisch MDI) im stationären Zustand; vgl. auch FIG. 5

4,3 t/h eines Gemisches aus Methylendiphenyldiamin und Polymethylenpolyphenylenpolyamin (nachfolgend summarisch MDA; 2) einer Temperatur von 110 °C werden mit 11 t/h Monochlorbenzol (MCB; 4) einer Temperatur von 30 °C als Lösungsmittel über einen statischen Mischer (5-1100) zu einer 28%igen MDA-Lösung (20) vermischt. Phosgen (3) wird bereitgestellt über einen Phosgengenerator und einen Phosgenverflüssiger. Im Anschluss wird das Phosgen (3) in einem Phosgenlösungstank mit MCB (4) auf eine 35%ige Phosgenlösung (30) eingestellt. Stündlich werden 24 Tonnen 35%ige Phosgenlösung (30) einer Temperatur von 0 °C mit 4,3 Tonnen MDA (2) in Form der 28%igen MDA-Lösung (20) einer Temperatur von 45 °C in einer adiabaten Reaktion, wie in EP 1 873 142 B1 beschrieben, umgesetzt. Nach der Durchmischung der beiden Rohstofflösungen im Mischaggregat (5-1100) wird die erhaltene Reaktionslösung (5) mit einer Temperatur von 85 °C über eine Suspensionsleitung (5-1110) in einen beheizten Phosgenierturm (5-1200) gefahren. Am Kopf des Phosgenierturmes liegen ein absoluter Druck von 1,6 bar und eine Temperatur von 111 °C vor. Der bei der Reaktion entstandene Chlorwasserstoff wird zusammen mit Spuren an Phosgen und MCB als Gasstrom (71) abgeführt. Das flüssige Reaktionsgemisch (61) wird dem Phosgenierturm (5-1200) entnommen und der Aufarbeitungssequenz (5-2000) zugeführt. Dazu wird es zunächst als Seitenstrom in eine beheizte Entphosgenierkolonne eingeleitet. Bei einer Kopftemperatur von 116 °C und einem absoluten Druck von 1,6 bar wird über Kopf Phosgen mit Spuren MCB und Chlorwasserstoff abgeführt. Phosgen wird in einer Phosgenabsorptionskolonne absorbiert und in den Phosgenlösungstank gefahren, und Chlorwasserstoff wird in einen Chlorwasserstoffabsorber und dann zur weiteren Verwendung in einen Salzsäuretank geleitet. Nach Abtrennung von Chlorwasserstoff und überschüssigem Phosgen aus der Isocyanat enthaltenden Reaktionslösung wird eine Isocyanat-Rohlösung erhalten, die aus dem Sumpf der Entphosgenierkolonne ausgetragen und mit einer Temperatur von 155 °C in eine erste Destillationsstufe gefahren wird, um sie vom Lösungsmittel MCB zu befreien. Der absolute Druck am Kopf dieser Lösungsmitteldestillationskolonne beträgt 800 mbar bei einer Sumpftemperatur von 155 °C. Über Kopf wird MCB gasförmig abgezogen, wobei dieser MCB-Gasstrom mit kaltem MCB (30 °C) in einer Waschkolonne besprüht wird, um einen möglichen Mitriss von Isocyanat in die Vakuumleitungen zu verhindern. Das Reaktionsprodukt wird aus dem Sumpf der Kolonne ausgetragen und in einer zweiten Kolonne bis auf 1 % von restlichem MCB befreit. Anschließend wird in einem Gegenstromverdampfer bei einem absoluten Druck von 20 mbar und einer Sumpftemperatur von 210 °C das Produkt von Nebenkomponenten wie Phenylisocyanat und restlichem MCB befreit. Dabei fallen als Sumpfprodukt 5,4 t/h MDI an, das mittels weiterer Destillation zu MDI der gewünschten Reinheit (1) aufgearbeitet und anschließend zur weiteren Verwendung in einen Tank gefahren wird.

So hergestelltes MDI hat einen Restlösemittelgehalt an MCB von < 5 ppm (GC), einen Gehalt an leicht hydrolysierbarem Chlor (auch als Acidität bezeichnet) von < 100 ppm (acidimetrische Titration gemäß Bayer-Analysenmethode 2011-0461102-96) und einen Gehalt von chemisch gebundenem Chlor von < 2000 ppm (Wickboldt-Verbrennung).

### Beispiel 1 (Vergleichsbeispiel, Senkung der Produktionskapazität von Nennkapazität auf 50 % der Nennkapazität)

*In diesem Beispiel wird die Zufuhr von Phosgen vor der Zufuhr von Amin reduziert, wodurch sich in der Übergangsperiode im Vergleich zum stationären Zustand der Nennkapazität der Phosgenüberschuss erniedrigt und die Konzentration des Amins in der Reaktionsmischung erhöht (d. h. die Masse an Lösungsmittel relativ zur Gesamtmasse der Reaktionsmischung ist erniedrigt).*

Die Herstellung von 5,4 t/h MDI in kontinuierlicher Fahrweise wurde, wie in den allgemeinen Bedingungen beschrieben, bei Nennkapazität durchgeführt. Um die Phosgenier-Anlage auf Halblast zu bringen, wurde die Phosgen-Lösungszufuhr 10 Sekunden vor der MDA-Lösungszufuhr reduziert und die beiden Mengenströme innerhalb von 40 Minuten stufenlos auf 50 % der Nennkapazität heruntergefahren. Bei Erreichen der Halblast wurden 2,15 t/h MDA als 28%ige Lösung in Monochlorbenzol und 12 t/h an 35%iger Phosgenlösung in den Mischer gefahren. Nach Aufarbeitung und Destillation des Roh-Isocyanates verblieben 2,7 t/h an Rein-MDI. Nach fünf Stunden kontinuierlicher Fahrweise bei Halblast musste die Phosgenieranlage komplett abgestellt werden, weil die Verteilerböden der Entphosgenierkolonne zu verstopften begannen und dadurch der Druckverlust über die Kolonne anstieg. Die Anlage musste abgefahren werden, um die Entphosgenierkolonne von angebackenem und lose in der Kolonne liegendem Harnstoff zu befreien bevor die Anlage wieder angefahren werden konnte.

### Beispiel 2 (erfindungsgemäß, Senkung der Produktionskapazität von Nennkapazität auf 50 % der Nennkapazität)

*In diesem Beispiel wird der Aminstrom zuerst reduziert, und anschließend wird zeitversetzt der Phosgenstrom reduziert, wobei bis zum Erreichen der "Halblastfahrweise" ein erhöhter Phosgenüberschuss und eine erniedrigte Aminkonzentration (d. h. eine erhöhte Lösungsmittelmasse relativ zur Gesamtmasse der Reaktionsmischung) vorliegen. Nach Erreichen der "Halblast" sind der Phosgenüberschuss und die Aminkonzentration auf dem gleichen Niveau wie vorher im stationären Zustand der Nennkapazität. Anschließend wird die Anlage wieder auf Nennkapazität hochgefahren, wobei bis zum Erreichen der Nennkapazität-Fahrweise ein erhöhter Phosgenüberschuss und eine erniedrigte Aminkonzentration (d. h. eine erhöhte Lösungsmittelmasse relativ zur Gesamtmasse der Reaktionsmischung) vorliegen. Nach Erreichen der Nennkapazität sind der Phosgenüberschuss und die Aminkonzentration auf dem gleichen Niveau wie vorher im stationären Zustand der Halblast-Fahrweise.*

Die Herstellung von 5,4 t/h MDI in kontinuierlicher Fahrweise wurde, wie in den allgemeinen Bedingungen beschrieben, bei Nennkapazität durchgeführt. Wegen Rohstoffmangels musste der Produktionsplan korrigiert werden. Dazu wurde der Phosgengenerator auf die benötigte Menge an Phosgen heruntergefahren. Die Phosgenier-Anlage wurde eine Minute später auf Halblast heruntergefahren, indem zuerst die MDA-Zufuhr reduziert wurde. Die Phosgen-Zufuhr zum Mischapparat lief für eine Minute mit der vorab laufenden Nennkapazitätsmenge weiter. Anschließend wurde die Phosgen-Zufuhr ebenfalls heruntergefahren, und nach 41 Minuten waren die beiden Mengenströme stufenlos auf eine Produktionskapazität von 50 % der Nennkapazität (Halblast) gebracht, entsprechend 2,15 t/h MDA als 28%ige Lösung in Monochlorbenzol und 12 t/h an 35%iger Phosgenlösung in Monochlorbenzol. Dies entsprach einer Produktionsleistung von 2,7 t/h (MDI). Die Anlage wurde bis zum Ende des Rohstoffmangels aus den Beständen des Rohstoffes im Tanklager gefahren. Der Rohstoffmangel war nach drei Tagen aufgehoben und die Anlage konnte wieder auf Nennkapazität gebracht werden, indem zuerst die Phosgen-Zufuhr erhöht wurde und anschließend nach 1 Minute die Amin-Zufuhr erhöht wurde. Nach 41 Minuten waren die beiden Mengenströme stufenlos auf Nennkapazität gebracht. Anschließend wurde die Phosgenieranlage, wie in den allgemeinen Bedingungen beschrieben, für mehrere Monate betrieben. Es gab keine Probleme mit Anbackungen oder Niederschlägen. Währen der ganzen Zeit, vor, während und nach der Halblast und in den beiden Übergangsphasen wurde spezifikationsgerechte Ware hergestellt.

Wie die Beispiele zeigen, entstehen bei einem unsachgemäßen Lastwechsel unmittelbar Niederschläge in der Phosgenieranlage der Flüssigphasenphosgenierung. Bei erfindungsgemäßer Vorgehensweise beim Herunter- und Hochfahren der Phosgenierung wird demgegenüber die Bildung von Anbackungen und Niederschlägen deutlich reduziert, die Anlage kann über einen langen Produktionszyklus, auch nach mehreren Lastwechseln, betrieben werden, und es entsteht während der ganzen Zeit spezifikationsgerechte Ware.

### Allgemeine Bedingungen für die Herstellung von TDI im stationären Zustand; vgl. auch FIG. 6

TDA (2) wird in einem Aminverdampfer (6-2000) gemeinsam mit Stickstoff (4) kontinuierlich verdampft. Der so erhaltene Amingasstrom (20), enthaltend 12 t/h gasförmiges TDA (2), wird über eine Leitung (2100), an deren zum Phosgenierungsreakor (6-3000) hin gelegenen Ende sich eine Amindüse befindet, kontinuierlich in den Phosgenierungsreaktor (6-3000) eingedüst. Die Verweilzeit des TDA-Stroms (20) von Verlassen des Verdampfers (6-2000) bis zum Austritt aus der Amindüse beträgt dabei 5 Sekunden. Gleichzeitig werden über einen Phosgengleichrichter, der, wie in EP-A-1 362 847 offenbart, eingesetzt wird, 61 t/h eines gasförmigen Phosgenstroms kontinuierlich in den Phosgenierungsreaktor (6-3000) eingedüst. Das eingesetzte Phosgen ist eine Mischung aus frischem Phosgen (3') und im Aufarbeitungsteil (6-5000) zurückgewonnenem Phosgen (3"). Dabei werden die beiden Edukte gut vermischt, und eine Rückvermischung unterbleibt. Die Temperatur des gasförmigen TDA-Stroms (20) am Düsenmund beträgt 380 °C (TDA hat ca. 1 Sekunde Verweilzeit bei dieser Temperatur im Zulauf zum Düsenmund). Das gasförmige Phosgen (30) hat eine Temperatur von 320 °C beim Verlassen des Phosgengleichrichters, wobei die Verweilzeit des heißen Phosgens zwischen letztem Phosgenüberhitzer und Phosgengleichrichter 2 Sekunden beträgt. Das Gemisch aus dem gasförmigen Aminstrom und dem gasförmigen Phosgenstrom hat eine Verweilzeit von 8 Sekunden im Gasphasenreaktor (6-3000) und reagiert bei einem absoluten Druck von 1692 mbar zu gasförmigem Reaktionsgemisch. Die sich anschließende Reaktionsabbruchzone (6-4000) umfasst eine zweistufige "Quenche", in welcher das gasförmige Reaktionsgemisch durch Einsprühen von ortho-Dichlorbenzol (ODB) auf 168 °C abgekühlt wird, sodass es kondensiert und sich im Sumpfbehälter (6-4100) ein Gemisch (60) aus Roh-TDI und ODB ansammelt. Überschüssiges Phosgen, bei der Reaktion entstandener Chlorwasserstoff und Inerte werden unter diesen Bedingungen aus dem Sumpfbehälter (6-4100) weitestgehend entgast, wobei der Mitriss an TDI mittels Einbauten vermindert wird. Dieser Prozessrestgasstrom (50) wird zur Wiedergewinnung von mitgerissenem TDI, Phosgen und Chlorwasserstoff, wie in WO 2011/003532, Seite 11, Zeile 24 bis 25 beschrieben, aufgearbeitet (*6*-5100). Das Gemisch (60) aus dem Sumpfbehälter (6-4100) wird, wie in EP 1 413 571 B1 beschrieben, aufgearbeitet (6-5200), wobei TDI (1) in einem Mengenstrom von 15,6 t/h erhalten wird.

So hergestelltes TDI (1) hat typischerweise eine Reinheit von > 99,97 % (Gaschromatographie, GC), einen Restlösemittelgehalt an ODB von < 5 ppm (GC), einen Restchlorgehalt von hydrolisierbarem Chlor von < 10 ppm (Titration gemäß ASTM D4663), eine Acidität von gebundenem Chlor < 5 ppm (Titration gemäß ASTM D5629), und die Farbzahl, gemessen als Hazenzahl, ist < 15 (bestimmt nach DIN EN ISO 6271).

### Beispiel 3 (Vergleichsbeispiel, Senkung der Produktionskapazität von Nennkapazität auf 50 % der Nennkapazität)

*In diesem Beispiel wird die Zufuhr von Phosgen vor der Zufuhr von Amin reduziert, wodurch sich in der Übergangsperiode im Vergleich zum stationären Zustand der Nennkapazität der Phosgenüberschuss erniedrigt und die Konzentration des Amins in der Reaktionsmischung erhöht (d. h. die Masse an Lösungsmittel relativ zur Gesamtmasse der Reaktionsmischung ist erniedrigt).*

Die Gasphasenanlage (6-10000) wird, wie in den allgemeinen Bedingungen zur Herstellung von TDI beschrieben, bei einer Produktionskapazität von 15,6 t/h an TDI bei Nennkapazität betrieben und dann wie folgt auf Halblast heruntergefahren: Um die Phosgenier-Anlage auf Halblast zu bringen, wurde die Phosgen-Lösungszufuhr 10 Sekunden vor der MDA-Lösungszufuhr reduziert und die beiden Mengenströme innerhalb von 40 Minuten stufenlos auf 50 % der Nennkapazität heruntergefahren.

Der Phosgengenerator wird heruntergefahren auf die benötigte Menge an Phosgen. Eine Minute später werden die Phosgenzufuhr zum Phosgenierreaktor (6-3000) reduziert und weitere 10 Sekunden später wird die Aminzufuhr reduziert. Anschließend werden die beiden Mengenströme innerhalb von 25 Minuten stufenlos auf 50 % der Nennkapazität heruntergefahren. Der Amingasstrom enthält nun 6 t/h gasförmiges TDA. Die Verweilzeit des TDA-Stroms (20) von Verlassen des Verdampfers (6-2000) bis zum Austritt aus der Amindüse beträgt dabei 10 Sekunden. Über den Phosgengleichrichter werden 30,5 t/h eines gasförmigen Phosgenstromes in den Phosgenierungsreaktor eingedüst, wobei die Verweilzeit des heißen Phosgens zwischen letztem Phosgenüberhitzer und Phosgengleichrichter 4 Sekunden beträgt. Nach Aufarbeitung und Destillation des Roh-Isocyanates verbleiben 7,8 t/h an Rein-TDI. Nach 2 Tagen muss die Anlage abgestellt werden, weil der Differenzdruck von Eintritt der Edukte TDA-Gasstrom und Phosgen-Gasstrom in den Phosgenierreaktor (6-3000) über den Brüdengasaustritt am Sumpf der Reaktionsabbruchzone (6-4000) bis zur TDI-Auswaschkolonne (*6*-5110) über die Zeit auf 823 mbar, statt 10 mbar bei Normalbetrieb, angestiegen ist, und die benötigte Energie zum Verdampfen von Phosgen und TDA und Überführung der Amin- und Phosgen-Gasströme in den Phosgenierreaktor (6-3000) kaum noch aufgebracht werden kann (die Siedetemperatur des TDA (2) limitiert bei zunehmendem Druck die Verdampferkapazität). Nach Abstellung und Öffnen der Anlage werden am Austritt der Amindüse, entlang der Oberfläche des Reaktorraumes und auf den Oberflächen der Quenchen Polyharnstoff-haltige massive Ablagerungen gefunden. Es erfolgt eine mehrtägige Reinigung der Anlage, bevor diese wieder angefahren werden kann.

### Beispiel 4 (erfindungsgemäß, Senkung der Produktionskapazität von Nennkapazität auf 50 % der Nennkapazität)

*In diesem Beispiel wird der Aminstrom zuerst reduziert, und anschließend wird zeitversetzt der Phosgenstrom reduziert wird. Dabei liegen bis zum Erreichen der "Halblastfahrweise" ein erhöhter Phosgenüberschusses und eine erniedrigte Aminkonzentration (d. h. eine erhöhte Lösungsmittelmasse relativ zur Gesamtmasse der Reaktionsmischung) vor. Nach Erreichen der Halblast sind der Phosgenüberschuss und die Aminkonzentration auf dem gleichen Niveau wie vorher im stationären Zustand der Nennkapazität. Anschließend wird die Anlage wieder auf Nennkapazität hochgefahren, wobei bis zum Erreichen der Nennkapazität-Fahrweise ein erhöhter Phosgenüberschusses und eine erniedrigte Aminkonzentration (d. h. eine erhöhte Lösungsmittelmasse relativ zur Gesamtmasse der Reaktionsmischung) vorliegen. Nach Erreichen der Nennkapazität sind der Phosgenüberschuss und die Aminkonzentration auf dem gleichen Niveau wie vorher im stationären Zustand der Halblast-Fahrweise.*

Die Gasphasenanlage (6-10000) wird, wie in den allgemeinen Bedingungen zur Herstellung von TDI beschrieben, bei einer Produktionskapazität von 15,6 t/h an TDI bei Nennkapazität betrieben und dann wie folgt auf Halblast heruntergefahren, 7 Tage bei Halblast betrieben und anschließend wieder auf Nennkapazität hochgefahren:
Wegen Rohstoffmangels musste der Produktionsplan korrigiert werden. Zuerst wird der Phosgengenerator auf die benötigte Menge an Phosgen heruntergefahren. Die Gasphasenannlage wird auf Halblast heruntergefahren, indem eine Minute später die TDA-Zufuhr zum Phosgenierreaktor (6-3000) reduziert und eine weitere Minute später die Phosgen-Zufuhr zum Phosgenierreaktor reduziert werden und beide Mengenströme werden dann innerhalb von 25 Minuten stufenlos auf 50 % der Nennkapazität heruntergefahren. Der Amingasstrom enthält nun 6 t/h gasförmiges TDA. Die Verweilzeit des TDA-Stroms (20) von Verlassen des Verdampfers (6-2000) bis zum Austritt aus der Amindüse beträgt dabei 10 Sekunden. Über den Phosgengleichrichter werden 30,5 t/h eines gasförmigen Phosgenstromes kontinuierlich in den Phosgenierungsreaktor eingedüst, wobei die Verweilzeit des heißen Phosgens zwischen letztem Phosgenüberhitzer und Phosgengleichrichter 4 Sekunden beträgt. Nach Aufarbeitung und Destillation des Roh-Isocyanates verbleiben 7,8 t/h an Rein-TDI. Die Gasphasenanlage wird bis zum Ende des Rohstoffmangels aus den Beständen des Rohstoffes im Tanklager gefahren. Nach 7 Tagen kann die Anlage wieder auf Nennkapazität gebracht werden. Dazu wird zuerst der Phosgengenerator auf die benötigte Menge an Phosgen hochgefahren. Die Nennkapazität wird erreicht, indem zuerst die Phosgen-Zufuhr erhöht wird, indem über den Phosgengleichrichter die 30,5 t/h eines gasförmigen Phosgenstroms von der Halblastfahrweise auf 61 t/h hochgezogen werden. 1 Minute nach Erhöhung der Phosgen-Zufuhr wird die Amin-Zufuhr erhöht. Nach 25 Minuten sind die beiden Mengenströme stufenlos auf Nennkapazität gebracht. Anschließend wird die Gasphasen-Phosgenieranlage, wie in den allgemeinen Bedingungen beschrieben, für mehrere Monate betrieben. Es gibt keine Probleme mit Anbackungen oder Niederschlägen. Währen der ganzen Zeit, vor, während und nach der Halblast und in den beiden Übergangsphasen ist spezifikationsgerechte Ware hergestellt worden.

Wie die Beispiele zeigen, entstehen bei einem unsachgemäßen Lastwechsel sofort Anbackungen an der Amindüse, im Phosgenierreaktor und den Oberflächen der Quenchen der Gasphasenphosgenierung. Bei erfindungsgemäßer Vorgehensweise beim Herunter- und Hochfahren der Phosgenierung wird demgegenüber die Bildung von Anbackungen und Niederschlägen deutlich reduziert. Die Anlage kann über einen langen Produktionszyklus, auch nach mehreren Lastwechseln, betrieben werden, und es entsteht während der ganzen Zeit spezifikationsgerechte Ware.

## Patentansprüche

1. Kontinuierlich betriebenes Verfahren zur Herstellung eines Isocyanats (**1**) mit einer angestrebten Produktionskapazität an Isocyanat (**1**), ausgedrückt als Massenstrom, von m_{1, Soll}, wobei mindestens einmal ein Wechsel der angestrebten Produktionskapazität m_{1, Soll} durchgeführt wird, umfassend die Umsetzung des zu dem Isocyanat (**1**) korrespondierenden Amins **(2)** mit Phosgen (3) in einem Reaktionsraum, bei dem
(i) das Amin **(2)** dem Reaktionsraum mit einem Massenstrom m₂ und Phosgen **(3)** dem Reaktionsraum mit einem Massenstrom m₃ zugeführt werden, wobei
(ii) m₂ und m₃ stets so gewählt werden, dass Phosgen **(3)** im Überschuss bezogen auf die primären Aminogruppen des Amins **(2)** vorliegt, wobei
(iii) bei dem mindestens einen Wechsel der angestrebten Produktionskapazität m_{1, Soll,} welcher von einem Ausgangszustand m_{1, Soll} (A) ≠ 0 mit einem korrespondierenden Amin-Massenstrom m_{2, Soll} (A) und einem unter Beachtung von (ii) ausgewählten Phosgen-Massenstrom m_{3, Soll} (A) auf einen Endzustand m_{1, Soll} (E) ≠ 0 mit einem korrespondierenden Amin-Massenstrom m_{2, Soll} (E) und einem unter Beachtung von (ii) ausgewählten Phosgen-Massenstrom m_{3, Soll} (E) durchgeführt wird, wobei der Wechsel der angestrebten Produktionskapazität m_{1, Soll} um einen Wert erfolgt, der im Bereich von 10 % bis 90 %, bezogen auf m_{1, Soll} (A), liegt, und wobei der Wechsel der angestrebten Produktionskapazität m_{1, Soll} von dem Ausgangszustand mi, _{Soll} (A) ≠ 0 auf den Endzustand m_{1, Soll} (E) ≠ 0 in einer Übergangsperiode t_{Ü} erfolgt, die mindestens 0,50 Minuten und maximal 3 Stunden beträgt,
die Massenströme an Amin **(2)** m₂ und Phosgen **(3)** m₃ während der Übergangsperiode zwischen Anfangs- und Endzustand so geändert werden, dass
(iii-1) der momentane Phosgenüberschuss zu dem Zeitpunkt der Übergangsperiode, bei dem mit der Änderung des Massenstroms m₂ von m_{2, Soll} (A) auf m_{2, Soll} (E) hin begonnen wird, mindestens genau so groß ist wie und bevorzugt größer ist als der Phosgenüberschuss während des Zeitraums der Produktion mit der Produktionskapazität m_{1, Soll} (A) vor Beginn der Übergangsperiode, und dass
(iii-2) der durchschnittliche Phosgenüberschuss während der Übergangsperiode größer ist als der Phosgenüberschuss während des Zeitraums der Produktion mit der Produktionskapazität m_{1, Soll} (A) vor Beginn der Übergangsperiode,
wobei der Phosgenüberschuss während der Produktion mit der Produktionskapazität m_{1, Soll} (A) vor Beginn der Übergangsperiode gleich dem Phosgenüberschuss während der Produktion mit der Produktionskapazität m_{1, Soll} (E) nach dem Ende der Übergangsperiode ist.

2. Verfahren gemäß Anspruch 1, bei dem m_{1,Soll}(A) größer ist als m_{1, Soll} (E), und bei dem mit der Reduzierung des dem Reaktionsraum zugeführten Amin-Massenstroms m₂ vor der Reduzierung des dem Reaktionsraum zugeführten Phosgen-Massenstroms m₃ begonnen wird.

3. Verfahren gemäß Anspruch 2, bei dem m₂ und m₃ in der Übergangsperiode so geändert werden, dass sie gleichzeitig die für den Endzustand angestrebten Werte m_{2, Soll} (E) und m_{3, Soll} (E) erreichen.

4. Verfahren gemäß Anspruch 2, bei dem m₂ und m₃ in der Übergangsperiode so geändert werden, dass m₂ vor m₃ den für den Endzustand jeweils angestrebten Wert m_{2, Soll} (E) bzw. m_{3, Soll} (E) erreicht.

5. Verfahren gemäß Anspruch 1, bei dem m_{1, Soll} (A) kleiner ist als m_{1, Soll} (E), und bei dem mit der Erhöhung des dem Reaktionsraum zugeführten Amin-Massenstroms m₂ nach der Erhöhung des dem Reaktionsraum zugeführten Phosgen-Massenstroms m₃ begonnen wird.

6. Verfahren gemäß Anspruch 5, bei dem m₂ und m₃ in der Übergangsperiode so geändert werden, dass sie gleichzeitig die für den Endzustand angestrebten Werte m₂, _{Soll} (E) und m_{3, Soll} (E) erreichen.

7. Verfahren gemäß Anspruch 5, bei dem m₂ und m₃ in der Übergangsperiode so geändert werden, dass m₂ nach m₃ den für den Endzustand jeweils angestrebten Wert m_{2, Soll} (E) bzw. m_{3, Soll} (E) erreicht.

8. Verfahren nach einem der Ansprüche 1 bis 7, bei dem die Umsetzung im Reaktionsraum in Gegenwart eines inerten Stoffes **(4)** stattfindet.

9. Verfahren nach Anspruch 8, bei dem
das Amin **(2)** dem Reaktionsraum in Form eines Gemisches **(20)** mit dem inerten Stoff **(4)** zugeführt wird, und/oder
bei dem das Phosgen **(3)** dem Reaktionsraum in Form eines Gemisches **(30)** mit dem inerten Stoff **(4)** zugeführt wird.

10. Verfahren nach Anspruch 9, bei dem die durchschnittliche Konzentration des Amins **(2)** im Gemisch **(20)** während der Übergangsperiode maximal gleich der durchschnittlichen Konzentration des Amins **(2)** im Gemisch **(20)** während des Zeitraums der Produktion mit der Produktionskapazität m_{1, Soll} (A) ist, und/oder
bei dem die durchschnittliche Konzentration des Phosgens **(3)** im Gemisch **(30)** während der Übergangsperiode gleich der durchschnittlichen Konzentration des Phosgen **(3)** im Gemisch **(30)** während des Zeitraums der Produktion mit der Produktionskapazität m_{1, Soll} (A) ist.

11. Verfahren gemäß einem der Ansprüche 9 oder 10, bei dem die Konzentration an Amin **(2)** im Gemisch **20** und die Konzentration an Phosgen **(3)** im Gemisch **30** nach der Übergangsperiode jeweils die gleiche ist wie vor der Übergangsperiode.

12. Verfahren gemäß einem der Ansprüche 1 bis 11, insbesondere gemäß einem der Ansprüche 8 bis 10, bei dem die Umsetzung des Amins **(2)** mit Phosgen **(3)** in der Flüssigphase stattfindet und bei dem insbesondere der Inertstoff **(4),** sofern vorhanden, ein inertes Lösungsmittel ist, und wobei das Isocyanat insbesondere ausgewählt ist aus der Gruppe bestehend aus
den Di- und Polyisocyanaten der Diphenylmethanreihe, Toluylendiisocyanat, Xylylendiisocyanat, Diisocyanatobenzol, 2,6-Xylolisocyanat, 1,5-Naphthylendiisocyanat, Diisocyanaten auf Basis von aliphatischen oder cycloaliphatischen Kohlenwasserstoffen mit 2 bis 18 Kohlenstoffatomen, wie insbesondere 1,4-Butandiisocyanat, 1,5-Pentandiisocyanat, 1,6-Hexandiisocyanat, 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat, 1,3- und 1,4-Cyclohexandiisocyanat, 1-Isocyanato-3,3,5-trimethyl-5-isocyanatomethylcyclohexan, 2,4-, oder 2,6-Diisocyanato-1-methylcyclohexan, 1-Isocyanato-1-methyl-4(3)-isocyanatomethylcyclohexan, 1,3 (und/oder 1,4)-Bis(isocyanatomethyl)cyclohexan, Bis(isocyanatomethyl)norbornan und 4,4'(und/oder 2,4')-Diisocyanatodicyclohexyl-methan
und
(cyclo)aliphatischen Triisocyanaten mit bis zu 22 Kohlenstoffatomen, wie insbesondere Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan und 1,3,5-Tris(isocyanatomethyl)-cyclohexan.

13. Verfahren gemäß einem der Ansprüche 1 bis 11, insbesondere gemäß einem der Ansprüche 8 bis 10, bei dem die Umsetzung des Amins **(2)** mit Phosgen **(3)** in der Gasphase stattfindet und bei dem insbesondere der Inertstoff **(4),** sofern vorhanden, ein inertes Gas ist, und wobei das Isocyanat insbesondere ausgewählt ist aus der Gruppe bestehend aus
den Diisocyanaten der Diphenylmethanreihe, Toluylendiisocyanat, Xylylendiisocyanat, Diisocyanatobenzol, 2,6-Xylolisocyanat, 1,5-Naphthylendiisocyanat, Diisocyanaten auf Basis von aliphatischen oder cycloaliphatischen Kohlenwasserstoffen mit 2 bis 18 Kohlenstoffatomen, wie insbesondere 1,4-Butandiisocyanat, 1,5-Pentandiisocyanat, 1,6-Hexandiisocyanat, 1,8-Octandiisocyanat, 1,9-Nonandiisocyanat, 1,10-Decandiisocyanat, 2,2-Dimethylpentan-1,5-diisocyanat, 2-Methyl-1,5-pentandiisocyanat (MPDI), 2,4,4(oder 2,2,4)-Trimethyl-1,6-hexandiisocyanat, 1,3- und 1,4-Cyclohexandiisocyanat, 1-Isocyanato-3,3,5-trimethyl-5-isocyanatomethylcyclohexan, 2,4-, oder 2,6-Diisocyanato-1-methylcyclohexan, 1-Isocyanato-1-methyl-4(3)-isocyanatomethylcyclohexan, 1,3 (und/oder 1,4)-Bis(isocyanatomethyl)cyclohexan, Bis(isocyanatomethyl)norbornan und 4,4'(und/oder 2,4')-Diisocyanatodicyclohexyl-methan
und
(cyclo)aliphatischen Triisocyanaten mit bis zu 22 Kohlenstoffatomen, wie insbesondere Triisocyanatocyclohexan, Tris(isocyanatomethyl)-cyclohexan, Triisocyanato-methylcyclohexan, 1,8-Diisocyanato-4-(isocyanatomethyl)octan, 1,6,11-Undecantriisocyanat, 1,7-Diisocyanato-4-(3-isocyanatopropyl)heptan, 1,6-Diisocyanato-3-(isocyanatomethyl)-hexan und 1,3,5-Tris(isocyanatomethy 1)-cyclohexan.

14. Verfahren gemäß einem der Ansprüche 1 bis 13, bei dem der Wechsel der angestrebten Produktionskapazität m_{1, Soll} um einen Wert erfolgt, der im Bereich von 20 % bis 80 %, bevorzugt im Bereich von 30 % bis 70 %, bezogen auf m_{1, Soll} (A), liegt.

15. Verfahren gemäß einem der Ansprüche 1 bis 14, bei welchem die Übergangsperiode mindestens 5,0 Minuten und maximal 3 Stunden beträgt.

## Claims

1. Continuous process for preparing an isocyanate **(1)** with a target production capacity of isocyanate **(1),** expressed as the mass flow rate, of m_{1,} target, with at least one change in the target production capacity m_{1,} target, comprising the reaction of the amine **(2)** corresponding to the isocyanate **(1)** with phosgene **(3)** in a reaction space, in which
(i) the amine **(2)** is fed to the reaction space with a mass flow rate m₂ and phosgene **(3)** to the reaction space with a mass flow rate m₃, wherein
(ii) m₂ and m₃ are always chosen such that phosgene **(3)** is present in excess relative to the primary amino groups of the amine **(2),** wherein
(iii) in the at least one change in the target production capacity m_{1,} target, which is conducted from a starting state m_{1, target} (A) ≠ 0 with a corresponding amine mass flow rate m₂, _{target} (A) and a phosgene mass flow rate m_{3, target} (A) selected with regard to (ii) to a final state m_{1, target} (E) # 0 with a corresponding amine mass flow rate m_{2, target} (E) and a phosgene mass flow rate m_{3, target} (E) selected with regard to (ii), where the change in the target production capacity m_{1, target} is by a value in the range from 10% to 90%, based on m_{1, target} (A), and where the change in the target production capacity m_{1, target} from the starting state m_{1, target} (A) ≠ 0 to the final state m_{1, target} (E) ≠ 0 is effected within a transition period t_{Ü} which is at least 0.50 minute and at most 3 hours,
the mass flow rates of amine **(2)** m₂ and phosgene **(3)** m₃ are altered during the transition period between the starting state and final state in such a way that
(iii-1) the instantaneous phosgene excess at the point in time in the transition period at which the alteration of the mass flow rate m₂ from m_{2, target} (A) to m_{2, target} (E) is commenced is at least as high as and preferably greater than the phosgene excess during the period of production with the production capacity m_{1, target} (A) prior to commencement of the transition period, and that
(iii-2) the average phosgene excess during the transition period is greater than the phosgene excess during the period of production with the production capacity m_{1, target} (A) prior to commencement of the transition period,
where the phosgene excess during the production with the production capacity m_{1, target} (A) prior to commencement of the transition period is equal to the phosgene excess during the production with the production capacity m_{1, target} (E) after the end of the transition period.

2. Process according to Claim 1, in which m_{1, target} (A) is greater than m_{1, target} (E), and in which the reduction in the amine mass flow m₂ fed to the reaction space is commenced prior to the reduction of the phosgene mass flow m₃ fed to the reaction space.

3. Process according to Claim 2, in which m₂ and m₃ are altered in the transition period such that they simultaneously attain the values m_{2, target} (E) and m_{3, target} (E) desired for the final state.

4. Process according to Claim 2, in which m₂ and m₃ are altered in the transition period such that m₂ attains the value m_{2, target} (E) desired for the final state before m₃ attains m_{3, target} (E) .

5. Process according to Claim 1, in which m_{1, target} (A) is less than m_{1, target} (E), and in which the increase in the amine mass flow m₂ fed to the reaction space is commenced after the increase in the phosgene mass flow m₃ fed to the reaction space.

6. Process according to Claim 5, in which m₂ and m₃ are altered in the transition period such that they simultaneously attain the values m₂, _{target} (E) and m_{3, target} (E) desired for the final state.

7. Process according to Claim 5, in which m₂ and m₃ are altered in the transition period such that m₂ attains the value m_{2, target} (E) desired for the final state after m₃ attains m_{3, target} (E) .

8. Process according to any of Claims 1 to 7, in which the reaction takes place in the reaction space in the presence of an inert substance **(4).**

9. Process according to Claim 8, in which
the amine **(2)** is fed to the reaction space **(20)** together with the inert substance **(4),** and/or
in which the phosgene **(3)** is fed to the reaction space **(30)** together with the inert substance **(4).**

10. Process according to Claim 9, in which the average concentration of the amine **(2)** in the mixture **(20)** during the transition period is not more than equal to the average concentration of the amine **(2)** in the mixture **(20)** during the period of production with the production capacity m_{1, target} (A), and/or
in which the average concentration of the phosgene **(3)** in the mixture **(30)** during the transition period is equal to the average concentration of the phosgene **(3)** in the mixture **(30)** during the period of production with the production capacity m_{1, target} (A) .

11. Process according to either of Claims 9 and 10, in which the concentration of amine **(2)** in the mixture **20** and the concentration of phosgene **(3)** in the mixture **30** after the transition period is the same in each case as prior to the transition period.

12. Process according to any of Claims 1 to 11, especially to any of Claims 8 to 10, in which the reaction of the amine **(2)** with phosgene **(3)** takes place in the liquid phase, and in which the inert substance **(4),** if present, is especially an inert solvent, and wherein the isocyanate is especially selected from the group consisting of
the di- and polyisocyanates of the diphenylmethane series, tolylene diisocyanate, xylylene diisocyanate, diisocyanatobenzene, xylene 2,6-isocyanate, naphthylene 1,5-diisocyanate, diisocyanates based on aliphatic or cycloaliphatic hydrocarbons having 2 to 18 carbon atoms, such as, more particularly, butane 1,4-diisocyanate, pentane 1,5-diisocyanate, hexane 1,6-diisocyanate, octane 1,8-diisocyanate, nonane 1,9-diisocyanate, decane 1,10-diisocyanate, 2,2-dimethylpentane 1,5-diisocyanate, 2-methyl-1,5-pentane diisocyanate (MPDI), 2,4,4(or 2,2,4)-trimethylhexane 1,6-diisocyanate, cyclohexane 1,3- and 1,4-diisocyanate, 1-isocyanato-3,3,5-trimethyl-5-isocyanatomethyl-cyclohexane, 2,4- or 2,6-diisocyanato-1-methylcyclohexane, 1-isocyanato-1-methyl-4(3)-isocyanatomethylcyclohexane, 1,3(and/or 1,4)-bis(isocyanatomethyl)cyclohexane, bis(isocyanatomethyl)norbornane and 4,4'(and/or 2,4')-diisocyanatodicyclohexylmethane
and
(cyclo)aliphatic triisocyanates having up to 22 carbon atoms, such as, more particularly, triisocyanatocyclohexane, tris(isocyanatomethyl)-cyclohexane, triisocyanatomethylcyclohexane, 1,8-diisocyanato-4-(isocyanatomethyl)octane, undecane 1,6,11-triisocyanate, 1,7-diisocyanato-4-(3-isocyanatopropyl)heptane, 1,6-diisocyanato-3-(isocyanatomethyl)hexane and 1,3,5-tris(isocyanatomethy1)cyclohexane.

13. Process according to any of Claims 1 to 11, especially to any of Claims 8 to 10, in which the reaction of the amine **(2)** with phosgene **(3)** takes place in the gas phase, and in which the inert substance **(4),** if present, is especially an inert gas, and wherein the isocyanate is especially selected from the group consisting of
the diisocyanates of the diphenylmethane series, tolylene diisocyanate, xylylene diisocyanate, diisocyanatobenzene, xylene 2,6-isocyanate, naphthylene 1,5-diisocyanate, diisocyanates based on aliphatic or cycloaliphatic hydrocarbons having 2 to 18 carbon atoms, such as, more particularly, butane 1,4-diisocyanate, pentane 1,5-diisocyanate, hexane 1,6-diisocyanate, octane 1,8-diisocyanate, nonane 1,9-diisocyanate, decane 1,10-diisocyanate, 2,2-dimethylpentane 1,5-diisocyanate, 2-methyl-1,5-pentane diisocyanate (MPDI), 2,4,4(or 2,2,4)-trimethylhexane 1,6-diisocyanate, cyclohexane 1,3-and 1,4-diisocyanate, 1-isocyanato-3,3,5-trimethyl-5-isocyanatomethylcyclohexane, 2,4- or 2,6-diisocyanato-1-methylcyclohexane, 1-isocyanato-1-methyl-4(3)-isocyanatomethylcyclohexane, 1,3(and/or 1,4)-bis(isocyanatomethyl)cyclohexane, bis(isocyanatomethyl)norbornane and 4,4'(and/or 2,4')-diisocyanatodicyclohexylmethane
and
(cyclo)aliphatic triisocyanates having up to 22 carbon atoms, such as, more particularly, triisocyanatocyclohexane, tris(isocyanatomethyl)-cyclohexane, triisocyanatomethylcyclohexane, 1,8-diisocyanato-4-(isocyanatomethyl)octane, undecane 1,6,11-triisocyanate, 1,7-diisocyanato-4-(3-isocyanatopropyl)heptane, 1,6-diisocyanato-3-(isocyanatomethyl)hexane and 1,3,5-tris(isocyanatomethyl)cyclohexane.

14. Process according to any of Claims 1 to 13, in which the change in the target production capacity m_{1,} target is by a value in the range from 20% to 80%, preferably in the range from 30% to 70%, based on m_{1, target} (A) .

15. Process according to any of Claims 1 to 14, in which the transition period is at least 5.0 minutes and at most 3 hours.

## Revendications

1. Procédé, exploité en continu, pour la préparation d'un isocyanate (1) à une capacité visée de production en isocyanate (1), exprimée comme flux massique, de m_{1,Consigne}, un changement de la capacité visée de production m_{1,Consigne} étant réalisé au moins une fois, comprenant la transformation de l'amine (2) correspondant à l'isocyanate (1) avec du phosgène (3) dans une chambre de réaction, dans laquelle
(i) l'amine (2) est introduite dans la chambre de réaction à un flux massique m₂ et le phosgène (3) est introduit dans la chambre de réaction à un flux massique m₃, où
(ii) m₂ et m₃ sont toujours choisis de manière telle que le phosgène (3) se trouve en excès par rapport aux groupes amino primaire de l'amine (2), où
(iii) lors dudit au moins un changement de la capacité visée de production m_{1,Consigne}, qui est réalisé à partir d'un état de départ m_{1,Consigne} (A) ≠ 0 avec un flux massique d'amine correspondant m_{2,Consigne} (A) et un flux massique de phosgène m_{3,Consigne} (A) choisi compte tenu de (ii) à un état final m_{1,Consigne} (E) ≠ 0 avec un flux massique d'amine correspondant m_{2,Consigne} (E) et un flux massique de phosgène m_{3,Consigne} (E) choisi compte tenu de (ii), le changement de la capacité visée de production m_{1,Consigne} étant réalisé autour d'une valeur qui se situe dans la plage de 10% à 90%, par rapport à m_{1,Consigne} (A), et le changement de la capacité visée de production m_{1,Consigne} à partir de l'état de départ m_{1,Consigne} (A) ≠ 0 à l'état final m_{1,Consigne} (E) ≠ 0 ayant lieu dans une période de transition t_{Ü} qui dure au moins 0,50 minute et au maximum 3 heures,
les flux massiques d'amine (2) m₂ et de phosgène (3) m₃ pendant la période de transition entre l'état de départ et l'état final étant modifiés de manière telle que
(iii-1) l'excès momentané de phosgène au moment de la période de transition où le changement du flux massique m₂ de m_{2,Consigne} (A) à m_{2,Consigne} (E) commence étant au moins aussi grand que l'excès de phosgène, et de préférence supérieur à celui-ci, pendant la période de production à la capacité de production m_{1,Consigne} (A) avant le début de la période de transition et que
(iii-2) l'excès moyen de phosgène pendant la période de transition est supérieur à l'excès de phosgène pendant la période de production à la capacité de production m_{1,Consigne} (A) avant le début de la période de transition,
l'excès de phosgène pendant la production à la capacité de production m_{1,Consigne} (A) avant le début de la période de transition étant identique à l'excès de phosgène pendant la production à la capacité de production m_{1,Consigne} (E) après la fin de la période de transition.

2. Procédé selon la revendication 1, dans lequel m_{1,Consigne} (A) est supérieur à m_{1,Consigne} (E) et dans lequel la réduction du flux massique d'amine m₂ introduit dans la chambre de réaction démarre avant la réduction du flux massique de phosgène m₃ introduit dans la chambre de réaction.

3. Procédé selon la revendication 2, dans lequel m₂ et m₃ sont modifiés dans la période de transition de manière telle qu'ils atteignent simultanément les valeurs m_{2,Consigne} (E) et m_{3,Consigne} (E) visées pour l'état final.

4. Procédé selon la revendication 2, dans lequel m₂ et m₃ sont modifiés dans la période de transition de manière telle que m₂ atteint avant m₃ la valeur respective m_{2,Consigne} (E) et m_{3,Consigne} (E) visée pour l'état final.

5. Procédé selon la revendication 1, dans lequel m_{1,Consigne} (A) est inférieur à m_{1,Consigne} (E) et dans lequel l'augmentation du flux massique d'amine m₂ introduit dans la chambre de réaction démarre après l'augmentation du flux massique de phosgène m₃ introduit dans la chambre de réaction.

6. Procédé selon la revendication 5, dans lequel m₂ et m₃ sont modifiés dans la période de transition de manière telle qu'ils atteignent simultanément les valeurs m_{2,Consigne} (E) et m_{3,Consigne} (E) visées pour l'état final.

7. Procédé selon la revendication 5, dans lequel m₂ et m₃ sont modifiés dans la période de transition de manière telle que m₂ atteint après m₃ la valeur respective m_{2,Consigne} (E) et m_{3,Consigne} (E) visée pour l'état final.

8. Procédé selon l'une quelconque des revendications 1 à 7, dans lequel la transformation dans la chambre de réaction a lieu en présence d'une substance inerte (4).

9. Procédé selon la revendication 8, dans lequel l'amine (2) est introduite dans la chambre de réaction sous forme d'un mélange (20) avec la substance inerte (4) et/ou dans lequel le phosgène (3) est introduit dans la chambre de réaction sous forme d'un mélange (30) avec la substance inerte (4).

10. Procédé selon la revendication 9, dans lequel la concentration moyenne en amine (2) dans le mélange (20) pendant la période de transition est au maximum identique à la concentration moyenne en amine (2) dans le mélange (20) pendant la période de production à la capacité de production m_{1,Consigne} (A) et/ou dans lequel la concentration moyenne en phosgène (3) dans le mélange (30) pendant la période de transition est identique à la concentration moyenne en phosgène (3) dans le mélange (30) pendant la période de production à la capacité de production m_{1,Consigne} (A) .

11. Procédé selon l'une quelconque des revendications 9 ou 10, dans lequel la concentration en amine (2) dans le mélange (20) et la concentration en phosgène (3) dans le mélange (30) après la période de transition est à chaque fois identique à celle avant la période de transition.

12. Procédé selon l'une quelconque des revendications 1 à 11, en particulier selon l'une quelconque des revendications 8 à 10, dans lequel la transformation de l'amine (2) avec du phosgène (3) a lieu en phase liquide et dans lequel en particulier la substance inerte (4), si elle est présente, est un solvant inerte et l'isocyanate étant en particulier choisi dans le groupe constitué par
- les diisocyanates et les polyisocyanates de la série du diphénylméthane, le diisocyanate de toluylène, le diisocyanate de xylylène, le diisocyanatobenzène, le 2,6-xylène-isocyanate, le 1,5-naphtylènediisocyanate, les diisocyanates à base d'hydrocarbures aliphatiques ou cycloaliphatiques comprenant 2 à 18 atomes de carbone, tels qu'en particulier le 1,4-butanediisocyanate, le 1,5-pentanediisocyanate, le 1,6-hexanediisocyanate, le 1,8-octanediisocyanate, le 1,9-nonanediisocyanate, le 1,10-décanediisocyanate, le 2,2-diméthylpentane-1,5-diisocyanate, le 2-méthyl-1,5-pentanediisocyanate (MPDI), le 2,4,4(ou 2,2,4)-triméthyl-1,6-hexanediisocyanate, le 1,3-cyclohexanediisocyanate et le 1,4-cyclohexanediisocyanate, le 1-isocyanato-3,3,5-triméthyl-5-isocyanatométhylcyclohexane, le 2,4-diisocyanato-1-méthylcyclohexane ou le 2,6-diisocyanato-1-méthylcyclohexane, le 1-isocyanato-1-méthyl-4(3)-isocyanatométhylcyclohexane, le 1,3(et/ou 1,4)-bis(isocyanatométhyl)cyclohexane, le bis(isocyanatométhyl)norbornane et le 4,4'(et/ou 2,4')-diisocyanatodicyclohexylméthane et
- les triisocyanates (cyclo)aliphatiques comprenant jusqu'à 22 atomes de carbone, tels qu'en particulier le triisocyanatocyclohexane, le tris(isocyanatométhyl)-cyclohexane, le triisocyanatométhylcyclohexane, le 1,8-diisocyanato-4-(isocyanatométhyl)octane, le 1,6,11-undécanetriisocyanate, le 1,7-diisocyanato-4-(3-isocyanatopropyl)heptane, le 1,6-diisocyanato-3-(isocyanatométhyl)-hexane et le 1,3,5-tris(isocyanatométhyl)-cyclohexane.

13. Procédé selon l'une quelconque des revendications 1 à 11, en particulier selon l'une quelconque des revendications 8 à 10, dans lequel la transformation de l'amine (2) avec du phosgène (3) a lieu en phase gazeuse et dans lequel en particulier la substance inerte (4), si elle est présente, est un gaz inerte et l'isocyanate étant en particulier choisi dans le groupe constitué par
- les diisocyanates de la série du diphénylméthane, le diisocyanate de toluylène, le diisocyanate de xylylène, le diisocyanatobenzène, le 2,6-xylène-isocyanate, le 1,5-naphtylènediisocyanate, les diisocyanates à base d'hydrocarbures aliphatiques ou cycloaliphatiques comprenant 2 à 18 atomes de carbone, tels qu'en particulier le 1,4-butanediisocyanate, le 1,5-pentanediisocyanate, le 1,6-hexanediisocyanate, le 1,8-octanediisocyanate, le 1,9-nonanediisocyanate, le 1,10-décanediisocyanate, le 2,2-diméthylpentane-1,5-diisocyanate, le 2-méthyl-1,5-pentanediisocyanate (MPDI), le 2,4,4(ou 2,2,4)-triméthyl-1,6-hexanediisocyanate, le 1,3-cyclohexanediisocyanate et le 1,4-cyclohexanediisocyanate, le 1-isocyanato-3,3,5-triméthyl-5-isocyanatométhylcyclohexane, le 2,4-diisocyanato-1-méthylcyclohexane ou le 2,6-diisocyanato-1-méthylcyclohexane, le 1-isocyanato-1-méthyl-4(3)-isocyanatométhylcyclohexane, le 1,3(et/ou 1,4)-bis(isocyanatométhyl)cyclohexane, le bis(isocyanatométhyl)norbornane et le 4,4'(et/ou 2,4')-diisocyanatodicyclohexylméthane et
- les triisocyanates (cyclo)aliphatiques comprenant jusqu'à 22 atomes de carbone, tels qu'en particulier le triisocyanatocyclohexane, le tris(isocyanatométhyl)-cyclohexane, le triisocyanatométhylcyclohexane, le 1,8-diisocyanato-4-(isocyanatométhyl)octane, le 1,6,11-undécanetriisocyanate, le 1,7-diisocyanato-4-(3-isocyanatopropyl)heptane, le 1,6-diisocyanato-3-(isocyanatométhyl)-hexane et le 1,3,5-tris(isocyanatométhyl)-cyclohexane.

14. Procédé selon l'une quelconque des revendications 1 à 13, dans lequel le changement de la capacité visée de production m_{1,Consigne} a lieu autour d'une valeur qui se situe dans la plage de 20% à 80%, de préférence dans la plage de 30% à 70%, par rapport à m_{1,Consigne} (A) .

15. Procédé selon l'une quelconque des revendications 1 à 14, dans lequel la période de transition dure au moins 5,0 minutes et au maximum 3 heures.
